# EUROPEAN PATENT APPLICATION

(11) **EP 4 723 281 A1**
(43) Date of publication of application: **08.04.2026**
(21) Application number: 23938989.3
(22) Date of filing: 02.06.2023
(51) Int. Cl.: H01M 10/0567, H01M 10/0525

(54) **NON-AQUEOUS ELECTROLYTE, SECONDARY BATTERY, AND ELECTRIC DEVICE**

(71) Applicant: Contemporary Amperex Technology Co., Limited, Ningde, Fujian 352100 (CN)
(72) Inventor: ZHANG, Limei, Ningde, Fujian 352100 (CN); CHEN, Peipei, Ningde, Fujian 352100 (CN); PENG, Chang, Ningde, Fujian 352100 (CN); LIU, Jiao, Ningde, Fujian 352100 (CN)
(74) Representative: Ziebig Hengelhaupt Intellectual Property Attorneys Patentanwaltskanzlei PartGmbB
(86) International application number: PCT/CN2023/098086
(87) International publication number: WO 2024/244005

(57) **Abstract**

Embodiments of the present application provide a non-aqueous electrolyte, a secondary battery and an electric device. The non-aqueous electrolyte comprises additives; the additives comprise a first additive and a second additive; the first additive is any one or more cyclic sulfate compounds having a structure represented by general formula (I); the second additive is an organic base additive; the organic base additive comprises any one or more of groups consisting of 5-12 membered aromatic heterocyclic organic bases or 5-12 membered aliphatic heterocyclic organic bases; ring structures in the 5-12 membered aromatic heterocyclic organic bases and the 5-12 membered aliphatic heterocyclic organic bases contain nitrogen atoms.

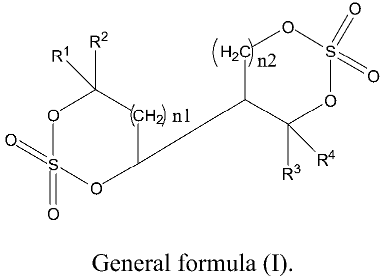

## Description

### TECHNICAL FIELD

The present application relates to the technical field of batteries, and in particular, to a non-aqueous electrolyte, a secondary battery and an electrical apparatus.

### BACKGROUND

For a lithium-ion battery, an increase in resistance and a decrease in capacity during high-temperature cycling and storage are serious performance degradation issues, and side reactions caused by the degradation of an electrolyte at high temperatures (especially the degradation caused by the decomposition of_{LiPF6}at high temperatures) are one of the causes of the above issues. HF generated by the decomposition of LiPF₆ may damage a solid electrolyte interface film (SEI film) on surfaces of positive and negative electrodes.

In particular, the negative electrode in an electrode material of the lithium-ion battery is a graphite-based negative electrode generally. For graphite, since its working potential is below 0.3V (relative to Li/Li⁺) (which is lower than an electrochemical stability window of an electrolyte used in the lithium-ion battery), the electrolyte used currently will be reduced and decomposed. Products of reduction and decomposition can form the SEI film, which inhibits further decomposition of the electrolyte. However, if the SEI film does not have a passivation capability sufficient to inhibit further decomposition of the electrolyte, the charged graphite self-discharges due to further decomposition of the electrolyte during storage, resulting in potential reduction of the entire battery.

One of factors that may affect the passivation capability of the SEI film is acids, such as HF and PF₅, generated by thermal decomposition of LiPF₆ (a lithium salt widely used in the lithtium-ion battery). When an electrode surface deteriorates due to acid attack, dissolution of transition metals occurs at the positive electrode, thereby increasing the resistance, and the capacity may be decreased due to the loss of a redox center. Because metal ions dissolved in this way will be electrodeposited on the negative electrode, the electron consumption caused by the electrodeposition of metal and the further decomposition of the electrolyte will lead to an increase in irreversible capacity. Accordingly, the battery capacity may decrease, the resistance may increase, and self-discharge of the graphite negative electrode may be caused.

### SUMMARY

The present application provides a non-aqueous electrolyte, a secondary battery and an electrical apparatus to improve cycling performance and storage performance of a battery.

A first aspect of the present application provides a non-aqueous electrolyte, including additives, wherein the additives include a first additive and a second additive; the first additive is any one or more cyclic sulfate compounds having a structure represented by general formula (I), 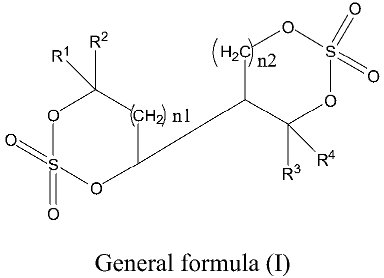
wherein R¹, R², R³, and R⁴ are each independently selected from any one of a group having a structure represented by general formula (II), a hydrogen atom, a halogen atom, a C1-C6 alkyl group, a C1-C6 haloalkyl group, a C1-C6 alkoxy group, a C1-C6 haloalkoxy group, a C2-C6 alkenyl group, a C2-C6 ester group, a cyano group, and a sulfonic acid group, and n1 and n2 are each independently any integer of 0-2,
the general formula (II) is 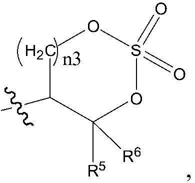
wherein R⁵ and R⁶ are each independently selected from any one of a group having a structure represented by the general formula (II), a hydrogen atom, a halogen atom, a C1-C6 alkyl group, a C1-C6 haloalkyl group, a C1-C6 alkoxy group, a C1-C6 haloalkoxy group, a C2-C6 alkenyl group, a C2-C6 ester group, a cyano group, and a sulfonic acid group, and n3 is any integer of 0-2; and
the second additive is an organic base additive, the organic base additive includes any one or more of groups consisting of 5-12 membered aromatic heterocyclic organic bases or 5-12 membered aliphatic heterocyclic organic bases, and ring structures of the 5-12 membered aromatic heterocyclic organic bases and the 5-12 membered aliphatic heterocyclic organic bases contain nitrogen atoms.

The cyclic sulfate in the non-aqueous electrolyte will form a good passivation film on the positive and negative electrode sides during the first charging process of the lithium-ion battery, reducing the damage to the positive and negative electrodes by acids produced by the decomposition of the lithium salt in the electrolyte, reducing the dissolution of the transition metal on the positive electrode side and the further decomposition of the electrolyte on the negative electrode side. Moreover, the non-aqueous electrolyte contains the above-mentioned second additive, which can effectively remove the acids produced by the decomposition of lithium salt in the electrolyte, further reduce the acidity of the electrolyte, and reduce the damage to the positive and negative electrode interfaces. The synergistic effect of the two additives can significantly reduce the damage of the acids produced by the decomposition of lithium salts to the positive and negative electrodes, thereby effectively alleviating the degradation of the solid electrolyte interface film or the dissolution of transition metals at the positive electrode during high-temperature cycling and storage, and effectively improving the battery cycling performance and storage performance.

In any embodiment of the first aspect, R¹ and R² are not both hydrogen atoms and R³ and R⁴ are not both hydrogen atoms.

In any embodiment of the first aspect, R¹, R², R³, R⁴, R⁵, and R⁶ satisfy the following conditions:
R¹ and R² are both hydrogen atoms, one of R³ and R⁴ is a hydrogen atom while the other is any one of a group having a structure represented by the general formula (II), a halogen atom, a C1-C6 alkyl group, a C1-C6 haloalkyl group, a C1-C6 alkoxy group, a C1-C6 haloalkoxy group, a C2-C6 alkenyl group, a C2-C6 ester group, a cyano group, and a sulfonic acid group, and R⁵ and R⁶ in the group having the structure represented by the general formula (II) are not both hydrogen atoms.

In any embodiment of the first aspect, R¹, R², R³, R⁴, R⁵, and R⁶ satisfy the following conditions:
R³ and R⁴ are both hydrogen atoms, one of R¹ and R² is a hydrogen atom while the other is any one of a group having a structure represented by the general formula (II), a halogen atom, a C1-C6 alkyl group, a C1-C6 haloalkyl group, a C1-C6 alkoxy group, a C1-C6 haloalkoxy group, a C2-C6 alkenyl group, a C2-C6 ester group, a cyano group, and a sulfonic acid group, and R⁵ and R⁶ in the group having the structure represented by the general formula (II) are not both hydrogen atoms.

In any embodiment of the first aspect, the cyclic sulfate compound has a structure represented by general formula (I-1), 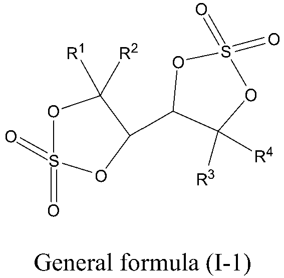
wherein R¹, R², R³ and R⁴ are each independently selected from any one of a group having a structure represented by general formula (II-1), a hydrogen atom, a halogen atom, a C1-C6 alkyl group, a C1-C6 haloalkyl group, a C1-C6 alkoxy group, a C1-C6 haloalkoxy group, a C2-C6 alkenyl group, a C2-C6 ester group, a cyano group, and a sulfonic acid group; and
the general formula (II-1) is 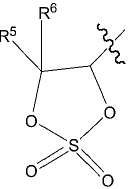
wherein R⁵ and R⁶ are each independently selected from any one of a group having a structure represented by the general formula (II-1), a hydrogen atom, a halogen atom, a C1-C6 alkyl group, a C1-C6 haloalkyl group, a C1-C6 alkoxy group, a C1-C6 haloalkoxy group, a C2-C6 alkenyl group, a C2-C6 ester group, a cyano group, and a sulfonic acid group.

In any embodiment of the first aspect, R¹, R², R³, R⁴, R⁵ and R⁶ are each independently selected from any one of a group having a structure represented by the general formula (II-1), a hydrogen atom, a halogen atom, a C1-C3 alkyl group, a C1-C3 haloalkyl group, a C1-C3 alkoxy group, a C1-C3 haloalkoxy group, a C2-C3 alkenyl group, a C1-C3 ester group, a cyano group, and a sulfonic acid group.

In any embodiment of the first aspect, R¹, R², R³, R⁴, R⁵ and R⁶ are each independently selected from any one of a group having a structure represented by the general formula (II-1), a hydrogen atom, a halogen atom, a C1-C3 alkyl group, and a C1-C3 haloalkyl group.

In any embodiment of the first aspect, R¹, R², R³, R⁴, R⁵ and R⁶ are each independently selected from any one of a group having a structure represented by the general formula (II-1), a hydrogen atom, an F atom, a Cl atom, a Br atom, a methyl group, an ethyl group, a propyl group, and an isopropyl group.

In any embodiment of the first aspect, the group having the structure represented by general formula (II-1) is selected from any one of the following groups: 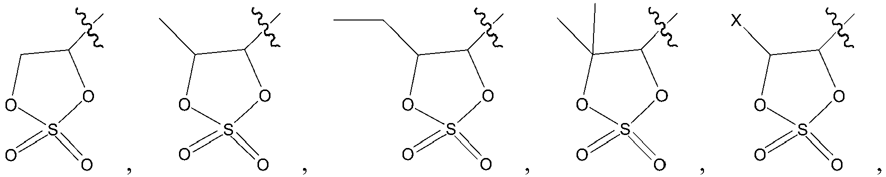 and 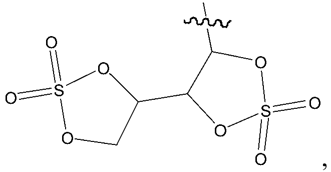 wherein X is an F atom, a Cl atom, or a Br atom.

In any embodiment of the first aspect, R¹, R², R³ and R⁴ are each independently selected from 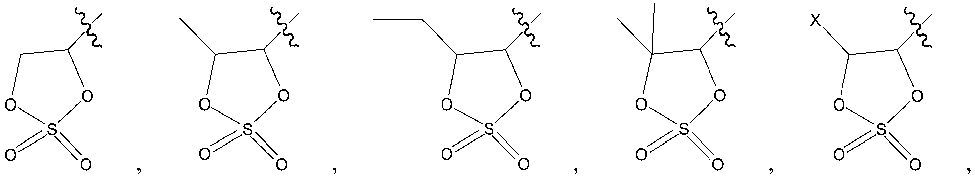 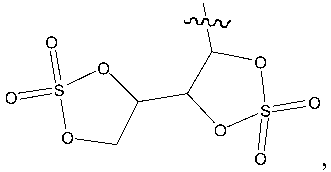 a hydrogen atom, an F atom, a Cl atom, a Br atom, a methyl group, an ethyl group, a propyl group, and an isopropyl group, and X is an F atom;

In any embodiment of the first aspect, R¹, R², R³ and R⁴ are each independently selected from 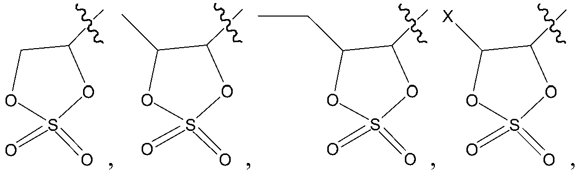 a hydrogen atom, a methyl group, and an ethyl group, and X is an F atom.

In any embodiment of the first aspect, the cyclic sulfate compound is selected from one or more of the following compounds:

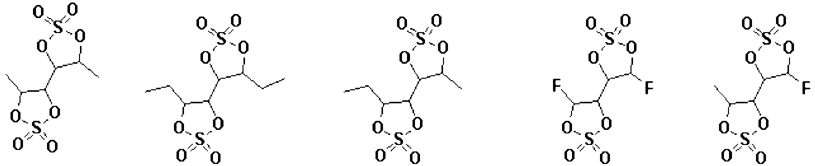

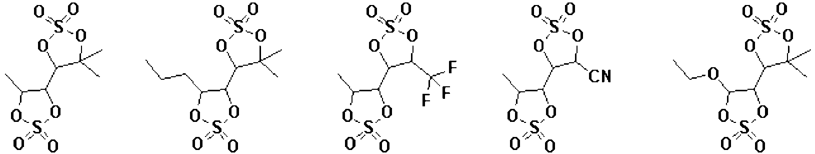

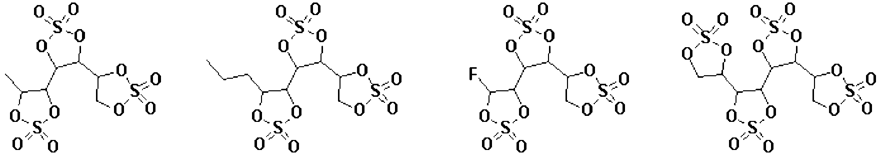

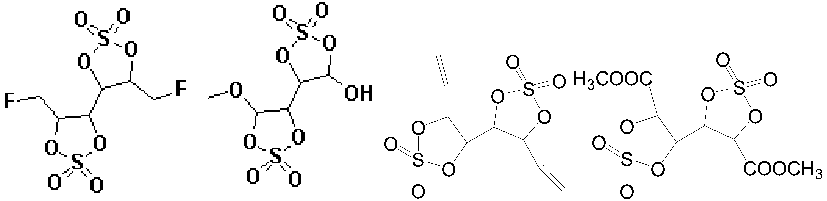

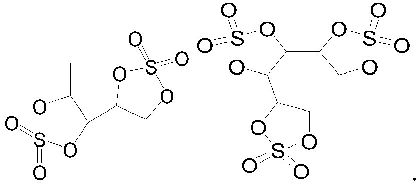

In any embodiment of the first aspect, the 5-12 membered aromatic heterocyclic organic bases include one or more selected from the group consisting of a compound having a structure represented by general formula (III), a compound having a structure represented by general formula (IV), and a compound having a structure represented by general formula (V), where 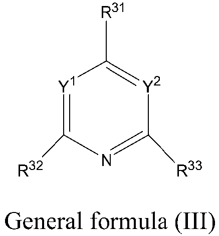 in the general formula (III), Y¹ and Y² are each independently a C or N element; R³¹, R³² and R³³ are each independently selected from any one of a hydrogen atom, a halogen atom, a C1-C6 alkyl group, a C2-C6 alkenyl group, a C2-C6 alkynyl group, -R³⁴OH, -R³⁵N R³⁶R³⁷ and-R³⁸-O-R³⁹; R³⁴, R³⁵ and R³⁸ are each independently selected from any one of a C0-C6 alkylene group and a C2-C6 alkenylene group; R³⁶, R³⁷ and R³⁹ are each independently selected from any one of a hydrogen atom, a halogen atom, a C1-C6 alkyl group and a C1-C6 haloalkyl group; any carbon atom in the C1-C6 alkyl group as the R³⁶, R³⁷ and R³⁹ is optionally substituted with a heteroatom; the heteroatom is an N atom, an S atom or a P atom; and optionally, the R³⁶ and R³⁷ are connected to form a ring; 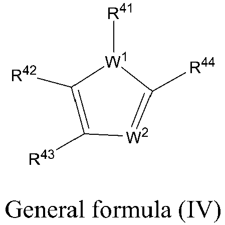 in the general formula (IV), W¹ is C, N, O or S; W² is C or N; at least one of W¹ and W² is N; R⁴¹, R⁴², R⁴³ and R⁴⁴ are each independently selected from any one of a hydrogen atom, a halogen atom, a C1-C6 alkyl group, a C2-C6 alkenyl group, a C2-C6 alkynyl group, -R⁴⁵OH, - R⁴⁶N R⁴⁷R⁴⁸ and -R⁴⁹-O-R⁵⁰; R⁴⁵, R⁴⁶ and R⁴⁹ are each independently selected from any one of a C0-C6 alkylene group and a C2-C6 alkenylene group; and R⁴⁷, R⁴⁸ and R⁵⁰ are each independently selected from any one of a hydrogen atom, a halogen atom, a C1-C6 alkyl group and a C1-C6 haloalkyl group; and 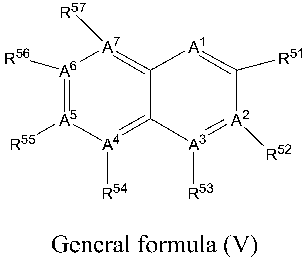 in the general formula (V), A¹, A², A³, A⁴, A⁵, A⁶ and A⁷ are each independently C or N; R⁵¹, R⁵², R⁵³, R⁵⁴, R⁵⁵, R⁵⁶ and R⁵⁷ are any one of a hydrogen atom, a C1-C6 alkyl group, a C2-C6 alkenyl group, a C2-C6 alkynyl group, -OH, -R⁵⁸N R⁵⁹R⁶⁰ and an alkoxy group; R⁵⁸is selected from any one of a C0-C6 alkylene group and a C2-C6 alkenylene group; and R⁵⁹ and R⁶⁰ are each independently selected from any one of a hydrogen atom, a halogen atom, a C1-C6 alkyl group and a C1-C6 haloalkyl group.

In any embodiment of the first aspect, in general formula (III), R³¹, R³² and R³³ are each independently selected from any one of a hydrogen atom, a halogen atom, a C1-C4 alkyl group, a C3-C5 alkenyl group, a C3-C5 alkynyl group, -R³⁴OH, -R³⁵N R³⁶R³⁷ and -R³⁸-O-R³⁹, R³⁴, R³⁵ and R³⁸are each independently selected from any one of C0-C3 alkylene groups, R³⁶, R³⁷ and R³⁹ are each independently selected from any one of a hydrogen atom, a halogen atom, a C1-C3 alkyl group and a C1-C3 haloalkyl group, any carbon atom in the C1-C6 alkyl group as the R³⁶, R³⁷ and R³⁹ is optionally substituted with a heteroatom, the heteroatom is an N atom, and optionally, the R³⁶ and R³⁷ are connected to form a 5-membered or 6-membered aliphatic heterocycle.

In any embodiment of the first aspect, R³¹, R³² and R³³ are each independently selected from any one of a hydrogen atom, a halogen atom, a methyl group, an ethyl group, an n-propyl group, an isopropyl group, an allyl group, a propargyl group, -OH, -CH₃OH, -NH₂, -CH₂NH₂, -N(CH₃)₂, O-CH₃ and 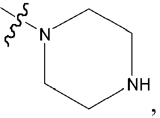 and optionally, the R³¹, R³² and R³³ are each independently selected from any one of a hydrogen atom, an F atom, a methyl group, an ethyl group, an n-propyl group, an isopropyl group, -CH₃OH, -CH₂NH₂, -N(CH₃)₂, O-CH₃ and

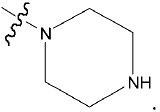

In any embodiment of the first aspect, the compound having the structure represented by general formula (III) is selected from any one or more of the following compounds:

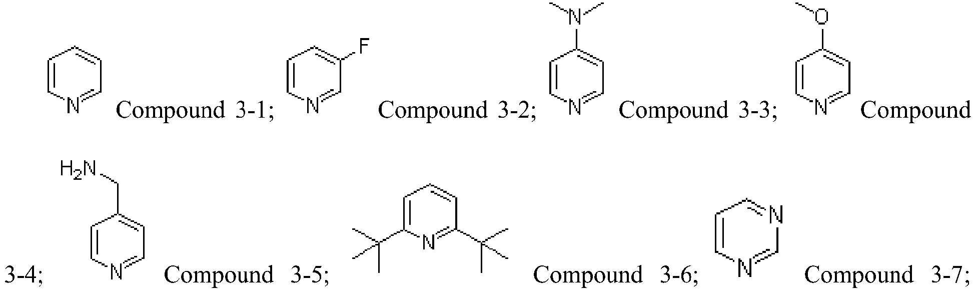

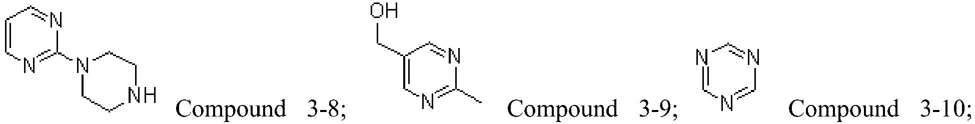

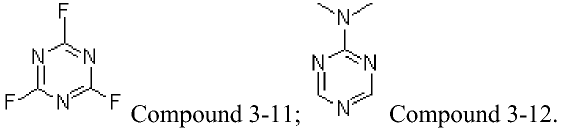

In any embodiment of the first aspect, in the general (IV), W¹ is N and W² is C or N.

In any embodiment of the first aspect, R⁴¹, R⁴², R⁴³ and R⁴⁴ are each independently selected from any one of a hydrogen atom, a halogen atom, a C1-C4 alkyl group, a C3-C5 alkenyl group, a C3-C5 alkynyl group, -R⁴⁴OH, -R⁴⁵N R⁴⁶R⁴⁷, and -R⁴⁸-O-R⁴⁹, R⁴⁴, R⁴⁵ and R⁴⁸ are each independently selected from any one of C0-C4 alkylene groups, and R⁴⁶, R⁴⁷ and R⁴⁹ are each independently selected from any one of a hydrogen atom, a halogen atom, a C1-C4 alkyl group and a C1-C4 haloalkyl group.

In any embodiment of the first aspect, R⁴¹, R⁴², R⁴³ and R⁴⁴ are each independently selected from any one of a hydrogen atom, an F atom, a methyl group, an ethyl group, a propyl group, a cyclopropyl group, an allyl group, a propargyl group, -OH, -CH₃OH, -NH₂, -NHCH₃, -CH₂NH₂, - N(CH₃)₂ and O-CH₃.

In any embodiment of the first aspect, R⁴¹, R⁴², R⁴³ and R⁴⁴ are each independently selected from any one of a hydrogen atom, an F atom, a methyl group, a propyl group, a cyclopropyl group, an allyl group, -CH₃OH, -NH₂, -NHCH₃ and -N(CH₃)₂.

In any embodiment of the first aspect, the compound having the structure represented by general formula (IV) is selected from any one or more of the following compounds:

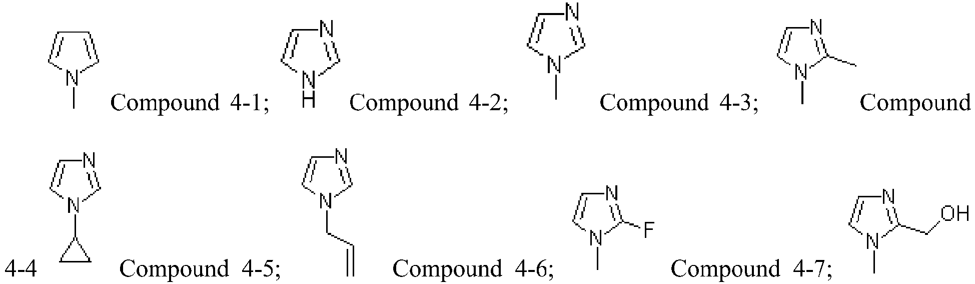

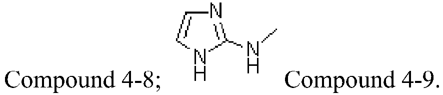

In any embodiment of the first aspect, in the general formula (V), A¹ is N, and A², A³, A⁴, A⁵, A⁶ and A⁷ are each independently C or N.

In any embodiment of the first aspect, R⁵¹, R⁵², R⁵³, R⁵⁴, R⁵⁵, R⁵⁶ and R⁵⁷ are any one of a hydrogen atom, a C1-C4 alkyl group, a C2-C4 alkenyl group, a C2-C4 alkynyl group, -OH, -R⁵⁸N R⁵⁹R⁶⁰ and an alkoxy group, R⁵⁸ is selected from any one of C0-C3 alkylene groups, and R⁵⁹ and R⁶⁰ are each independently selected from any one of a hydrogen atom, a halogen atom, a C1-C3 alkyl group and a C1-C3 haloalkyl group.

In any embodiment of the first aspect, R⁵¹, R⁵², R⁵³, R⁵⁴, R⁵⁵, R⁵⁶ and R⁵⁷ are any one of a hydrogen atom, a methyl group, an ethyl group, an allyl group, a propargyl group,-OH, -NH₂, - CH₂NH₂, -N(CH₃)₂ and O-CH₃.

In any embodiment of the first aspect, R⁵¹, R⁵², R⁵³, R⁵⁴, R⁵⁵, R⁵⁶ and R⁵⁷ are any one of a hydrogen atom, a methyl group, an ethyl group, and O-CH₃.

In any embodiment of the first aspect, the compound having the structure represented by general formula (V) is selected from any one or more of the following compounds:

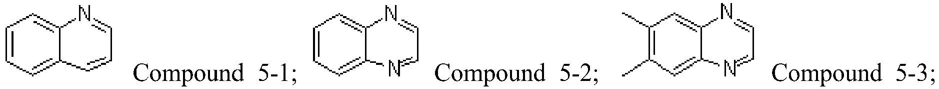

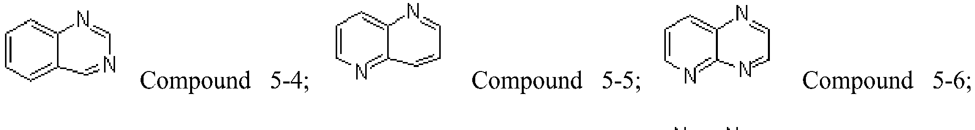

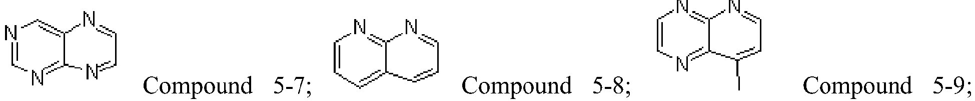

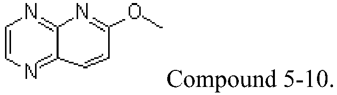

In any embodiment of the first aspect, the 5-12 membered aliphatic heterocyclic organic bases include one or more selected from the group consisting of a compound having a structure represented by general formula (VI) and a compound having a structure represented by general formula (VII), where 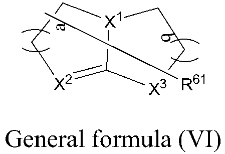 in the general formula (VI), X¹, X² and X³ are each independently C or N and at least one of them is required to be N; a and b are each independently an integer of 0-3; each R⁶¹ is independently selected from any one of a hydrogen atom, a halogen atom, a C1-C6 alkyl group, a C2-C6 alkenyl group, a C2-C6 alkynyl group, -R⁶²OH, -R⁶³N R⁶⁴R⁶⁵ and -R⁶⁶-O-R⁶⁷; R⁶², R⁶³ and R⁶⁴ are each independently selected from any one of a C0-C6 alkylene group and a C2-C6 alkenylene group; and R⁶⁵, R⁶⁶ and R⁶⁷ are each independently selected from any one of a hydrogen atom, a halogen atom, a C1-C6 alkyl group and a C1-C6 haloalkyl group, and 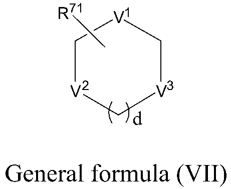 in the general formula (VI), V¹, V² and V³ are each independently C or N and at least one of them is required to be N; d is an integer of 0-3; each R⁷¹ is independently selected from any one of a hydrogen atom, a halogen atom, a C1-C6 alkyl group, a C2-C6 alkenyl group, a C2-C6 alkynyl group, -R⁷²OH, -R⁷³N R⁷⁴R⁷⁵ and -R⁷⁶-O-R⁷⁷; R⁷², R⁷³ and R⁷⁴ are each independently selected from any one of a C0-C6 alkylene group and a C2-C6 alkenylene group; and R⁷⁵, R⁷⁶ and R⁷⁷ are each independently selected from any one of a hydrogen atom, a halogen atom, a C1-C6 alkyl group and a C1-C6 haloalkyl group.

In any embodiment of the first aspect, in the general formula (VI), X¹ is N, and X² and X³ are each independently C or N.

In any embodiment of the first aspect, a and b are each independently 0, 1 or 2.

In any embodiment of the first aspect, each R⁶¹ is independently selected from any one of a hydrogen atom, a halogen atom, a C1-C4 alkyl group, a C3-C5 alkenyl group, a C3-C5 alkynyl group, -R⁶²OH, -R⁶³N R⁶⁴R⁶⁵, and -R⁶⁶-O-R⁶⁷, R⁶², R⁶³ and R⁶⁴ are each independently selected from any one of C0-C3 alkylene groups, and R⁶⁵, R⁶⁶ and R⁶⁷ are each independently selected from any one of a hydrogen atom, a halogen atom, a C1-C4 alkyl group and a C1-C4 haloalkyl group.

In any embodiment of the first aspect, each R⁶¹ is independently selected from any one of a hydrogen atom, an F atom, a methyl group, an ethyl group, a hydroxyl group, -NH₂, and -N(CH₃)₂.

In any embodiment of the first aspect, the compound having the structure represented by general formula (VI) is selected from any one or more of the following compounds:

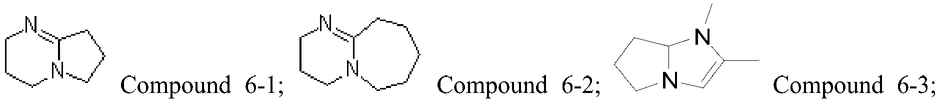

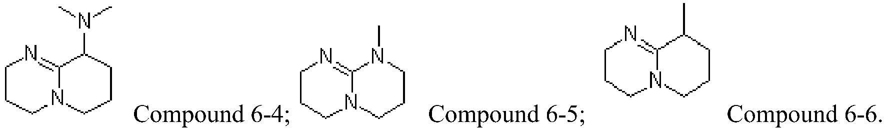

In any embodiment of the first aspect, in the general (VII), d is 0 or 1.

In any embodiment of the first aspect, each R⁷¹ is independently selected from any one of a hydrogen atom, a halogen atom, a C1-C4 alkyl group, a C3-C5 alkenyl group, a C3-C5 alkynyl group, -R⁷²OH, -R⁷³N R⁷⁴R⁷⁵, and -R⁷⁶-O-R⁷⁷, R⁷², R⁷³ and R⁷⁴ are each independently selected from any one of C0-C3 alkylene groups, and R⁷⁵, R⁷⁶ and R⁷⁷ are each independently selected from any one of a hydrogen atom, a halogen atom, a C1-C4 alkyl group and a C1-C4 haloalkyl group.

In any embodiment of the first aspect, each R⁷¹ is independently selected from any one of a hydrogen atom, an F atom, a methyl group, an ethyl group, an n-propyl group, an isopropyl group, a hydroxyl group, -NH₂, and -N(CH₃)₂.

In any embodiment of the first aspect, each R⁷¹ is independently selected from any one of a hydrogen atom, an F atom, a methyl group, an ethyl group, an n-propyl group, and an isopropyl group.

In any embodiment of the first aspect, the compound having the structure represented by general formula (VII) is selected from any one or more of the following compounds:

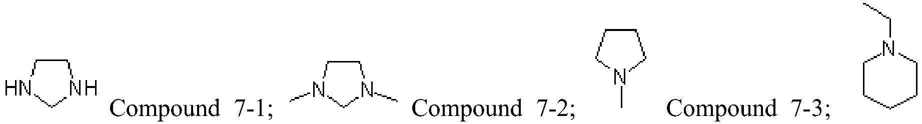

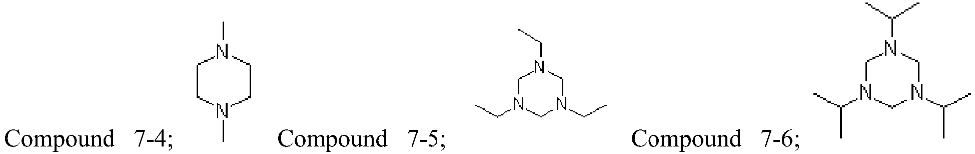

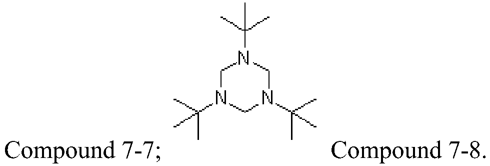

In any embodiment of the first aspect, mass percent of the second additive in the non-aqueous electrolyte is W1, W1 is optionally 0.001-20%, and W1 is further optionally 0.1-5%.

In any embodiment of the first aspect, percent of the second additive in the non-aqueous electrolyte is W2, W2 is optionally 0.001-20%, and W2 is further optionally 0.1-5%.

In any embodiment of the first aspect, 0.01≤W1/W2≤10, preferably 0.05≤W1/W2≤5.

In any embodiment of the first aspect, the non-aqueous electrolyte further includes an electrolyte; the electrolyte optionally includes an alkali metal salt electrolyte; the electrolyte optionally includes a lithium salt or a sodium salt; the lithium salt optionally includes one or more selected from the group consisting of lithium hexafluorophosphate, lithium perchlorate, lithium hexafluoroarsenate, lithium bis(fluorosulfonyl)imide, and lithium bis(trifluoromethanesulfonyl)imide; the sodium salt includes one or more selected from the group consisting of sodium hexafluorophosphate, sodium difluoro(oxalato)borate, sodium perchlorate, sodium bis(fluorosulfonyl)imide, sodium bis(trifluoromethanesulfonyl)imide, and sodium trifluoromethanesulfonate.

In any embodiment of the first aspect, the non-aqueous electrolyte further includes a non-aqueous solvent, the non-aqueous solvent optionally includes any one or more selected from the group consisting of cyclic carbonate, chain carbonate, a nitrile solvent, a ketone solvent, and a sulfone solvent, and the non-aqueous solvent further optionally includes one or more selected from the group consisting of ethylene carbonate, propylene carbonate, methyl ethyl carbonate, diethyl carbonate, dimethyl carbonate, dipropyl carbonate, methyl propyl carbonate, ethyl propyl carbonate, butylene carbonate, fluoroethylene carbonate, methyl formate, methyl acetate, ethyl acetate, propyl acetate, methyl propionate, ethyl propionate, propyl propionate, methyl butyrate, ethyl butyrate, 1,4-butyrolactone, sulfolane, dimethyl sulfone, methyl ethyl sulfone, diethyl sulfone, tetrahydrofuran, glycol dimethyl ether, dioxolane, acetone, acetonitrile, and butyronitrile.

In any embodiment of the first aspect, the additives further include one or more selected from the group consisting of a sulfate compound, a sulfite compound, a sultone compound, a disulfonic acid compound, a nitrile compound, an aromatic compound, an isocyanate compound, a phosphazene compound, a cyclic acid anhydride compound, a phosphite compound, a phosphate compound, a borate compound, and a carboxylate compound.

A second aspect of the present application provides a secondary battery, which includes a positive electrode plate, an electrolyte, a separator and a negative electrode plate, wherein the electrolyte includes any of the non-aqueous electrolytes in the first aspect, and optionally, the secondary battery is a lithium-ion secondary battery or a sodium-ion secondary battery.

In any embodiment of the second aspect, the negative electrode plate includes a negative electrode current collector and a negative electrode active material layer arranged on one side or both sides of the negative electrode current collector, and the negative electrode active material layer has a porosity of 30-45%, optionally 37-42%.

In any embodiment of the second aspect, the negative electrode active material layer includes a negative electrode active material; optionally, the negative electrode active material has D_{V}50≥6 µm; further optionally, the negative electrode active material has D_{V}50 between 15 µm and 20 µm.

A third aspect of the present application provides an electrical apparatus, including a secondary battery, the secondary battery including any of the secondary batteries as described in the second aspect.

### DESCRIPTION OF THE DRAWINGS

In order to illustrate the technical solutions of the Examples of the present application more clearly, the drawings required in the Examples of the present application will be briefly introduced below. Obviously, the drawings described below are only some Examples of the present application. For those of ordinary skill in the art, other drawings may also be obtained according to the drawings without any creative effort.
Fig. 1 is a schematic diagram of a secondary battery according to an embodiment of the present application;
Fig. 2 is an exploded view of the secondary battery according to an embodiment of the present application shown in Fig. 1;
Fig. 3 is a schematic diagram of a battery module according to an embodiment of the present application;
Fig. 4 is a schematic diagram of a battery pack according to an embodiment of the present application;
Fig. 5 is an exploded view of the battery pack according to an embodiment of the present application shown in Fig. 4;
Fig. 6 is a schematic diagram of an electrical apparatus using a secondary battery as a power source, according to an embodiment of the present application.

In the accompanying drawings, the figures are not necessarily drawn to actual scale.

### Description of reference numerals:

1. Battery pack; 2. Upper box; 3. Lower box; 4. Battery module; 5. Secondary battery; 51. Case; 52. Electrode assembly; 53. Top cover assembly.

### DETAILED DESCRIPTION

Embodiments of the present application are described in further detail below in conjunction with the drawings and embodiments. The following detailed description of the examples and the drawings are used to illustrate the principles of the present application by way of example, but should not be used to limit the scope of the present application, that is, the present application is not limited to the described examples.

Hereinafter, embodiments of a non-aqueous electrolyte, a secondary battery and an electrical apparatus of the present application are described in detail and specifically disclosed with reference to the drawings as appropriate. However, there may be cases where unnecessary detailed description is omitted. For example, there are cases where detailed descriptions of well-known items and repeated descriptions of actually identical structures are omitted. This is to avoid unnecessary redundancy in the following descriptions and to facilitate understanding by those skilled in the art. In addition, the drawings and subsequent descriptions are provided for those skilled in the art to fully understand the present application, and are not intended to limit the subject matter recited in the claims.

The "ranges" disclosed in the present application are defined in the form of lower and upper limits. A given range is defined by selecting a lower limit and an upper limit, and the selected lower and upper limits define the boundaries of the particular range. The range defined in this way may include or may not include end values, and may be arbitrarily combined, that is, any lower limit can be combined with any upper limit to form a range. For example, if the ranges 60-120 and 80-110 are listed for specific parameters, it is understood that the ranges 60-110 and 80-120 are also expected. In addition, if the listed minimum range values are 1 and 2 and if the listed maximum range values are 3, 4, and 5, the following ranges can all be expected: 1-3, 1-4, 1-5, 2-3, 2-4, and 2-5. In the present application, unless otherwise specified, the numerical range "a-b" represents an abbreviated representation of any combination of real numbers between a and b, wherein a and b are both real numbers. For example, the numerical range "0-5" indicates that all real numbers between "0-5" have been listed herein, and "0-5" is only a shortened representation of these numerical combinations. Additionally, when it is stated that a certain parameter is an integer of ≥2, it is equivalent to disclosing that the parameter is, for example, an integer of 2, 3, 4, 5, 6, 7, 8, 9, 10, 11, 12, etc.

Unless otherwise specified, all the embodiments and optional embodiments of the present application can be combined with each other form new technical solutions.

Unless otherwise specified, all technical features and optional technical features of the present application may be combined with each other to form new technical solutions.

Unless otherwise specified, all steps of the present application may be performed sequentially or randomly, and preferably sequentially. For example, the method includes steps (a) and (b), meaning that the method may include steps (a) and (b) performed sequentially, or may include steps (b) and (a) performed sequentially. For example, the reference to the method may further include step (c), meaning that step (c) may be added to the method in any order. For example, the method may include steps (a), (b) and (c), or may further include steps (a), (c) and (b), or may further include steps (c), (a) and (b), and the like.

Unless otherwise specifically stated, "including" and "comprising" mentioned in the present application indicate either open inclusion or closed inclusion. For example, the terms "including" and "comprising" may indicate that other components not listed may be further included or comprised, or only the listed components may be included or comprised.

Unless otherwise specifically stated, in the present application, the term "or" is inclusive. For example, the phrase "A or B" means "A, B, or both A and B." More specifically, the condition "A or B" is satisfied under any one of the following conditions: A is true (or present) and B is false (or absent); A is false (or absent) and B is true (or present); or both A and B are true (or present).

### [Secondary battery]

A secondary battery also known as a rechargeable battery or a storage battery refers to a battery that can be used continually by activating an active material in a charging manner after the battery is discharged.

Generally, the secondary battery includes a positive electrode plate, a negative electrode plate, a separator and an electrolyte. During charging and discharging of the battery, active ions (e.g., lithium ions) are intercalated and deintercalated back and forth between the positive electrode plate and the negative electrode plate. The separator is arranged between the positive electrode plate and the negative electrode plate, and mainly functions to prevent a short circuit between the positive electrode and the negative electrode while allowing active ions to pass through. The electrolyte mainly serves to conduct active ions between the positive electrode plate and the negative electrode plate.

### [Non-aqueous electrolyte]

In an embodiment of the present application, provided is a non-aqueous electrolyte, including additives, wherein the additives include a first additive and a second additive; the first additive is any one or more cyclic sulfate compounds having a structure represented by general formula (I), 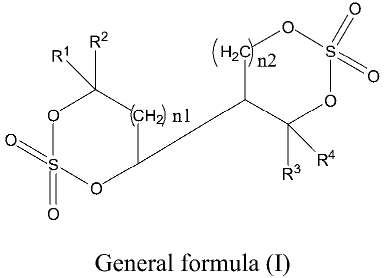
wherein R¹, R², R³, and R⁴ are each independently selected from any one of a group having a structure represented by general formula (II), a hydrogen atom, a halogen atom, a C1-C6 alkyl group, a C1-C6 haloalkyl group, a C1-C6 alkoxy group, a C1-C6 haloalkoxy group, a C2-C6 alkenyl group, a C2-C6 ester group, a cyano group, and a sulfonic acid group, and n1 and n2 are each independently any integer of 0-2,
the general formula (II) is 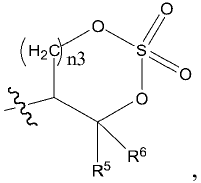
wherein R⁵ and R⁶ are each independently selected from any one of a group having a structure represented by the general formula (II), a hydrogen atom, a halogen atom, a C1-C6 alkyl group, a C1-C6 haloalkyl group, a C1-C6 alkoxy group, a C1-C6 haloalkoxy group, a C2-C6 alkenyl group, a C2-C6 ester group, a cyano group, and a sulfonic acid group, and n3 is any integer of 0-2; and
the second additive is an organic base additive, the organic base additive includes any one or more of groups consisting of 5-12 membered aromatic heterocyclic organic bases or 5-12 membered aliphatic heterocyclic organic bases, and ring structures of the 5-12 membered aromatic heterocyclic organic bases and the 5-12 membered aliphatic heterocyclic organic bases contain nitrogen atoms.

The cyclic sulfate in the non-aqueous electrolyte will form a good passivation film on the positive and negative electrode sides during the first charging process of the lithium-ion battery, reducing the damage to the positive and negative electrodes by acids produced by the decomposition of the lithium salt in the electrolyte, reducing the dissolution of the transition metal on the positive electrode side and the further decomposition of the electrolyte on the negative electrode side. Moreover, the non-aqueous electrolyte contains the above-mentioned second additive, which can effectively remove the acids produced by the decomposition of lithium salt in the electrolyte, further reduce the acidity of the electrolyte, and reduce the damage to the positive and negative electrode interfaces. The synergistic effect of the two additives can significantly reduce the damage of the acids produced by the decomposition of lithium salts to the positive and negative electrodes, thereby effectively alleviating the degradation of the solid electrolyte interface film or the dissolution of transition metals at the positive electrode during high-temperature cycling and storage, and effectively improving the battery cycling performance and storage performance.

In some embodiments, R¹ and R² are not both hydrogen atoms and R³ and R⁴ are not both hydrogen atoms. Of course, R¹, R², R³, and R⁴ may all be hydrogen atoms.

In some embodiments, R¹, R², R³, R⁴, R⁵, and R⁶ satisfy the following conditions:
R¹ and R² are both hydrogen atoms, one of R³ and R⁴ is a hydrogen atom while the other is any one of a group having a structure represented by the general formula (II), a halogen atom, a C1-C6 alkyl group, a C1-C6 haloalkyl group, a C1-C6 alkoxy group, a C1-C6 haloalkoxy group, a C2-C6 alkenyl group, a C2-C6 ester group, a cyano group, and a sulfonic acid group, and R⁵ and R⁶ in the group having the structure represented by the general formula (II) are not both hydrogen atoms.

In some embodiments, R¹, R², R³, R⁴, R⁵, and R⁶ satisfy the following conditions:
R³ and R⁴ are both hydrogen atoms, one of R¹ and R² is a hydrogen atom while the other is any one of a group having a structure represented by the general formula (II), a halogen atom, a C1-C6 alkyl group, a C1-C6 haloalkyl group, a C1-C6 alkoxy group, a C1-C6 haloalkoxy group, a C2-C6 alkenyl group, a C2-C6 ester group, a cyano group, and a sulfonic acid group, and R⁵ and R⁶ in the group having the structure represented by the general formula (II) are not both hydrogen atoms.

There are substituents in the above groups of R¹, R², R³, and R⁴. By introducing the substituents such as an alkyl group, a flexible SEI film with longer organic chains may be generated on the negative electrode, which can cope with a volume change of the negative electrode during cycling to avoid damage to the SEI film. The substituents such as the F-containing or N-containing substituent may be involved in film formation on the negative electrode, to generate an SEI film enriched with more inorganic components such as LiF and Li₃, thus increasing mechanical strength of the SEI film, improving stability of the SEI film on the negative electrode, and achieving the purpose of further improving the cycling performance of the battery.

The above-mentioned alkyl group may be a straight-chain alkyl group, a branched-chain alkyl group, or a cycloalkyl group, including but not limited to a methyl group, an ethyl group, a propyl group, an isopropyl group, an n-butyl group, an isobutyl group, a cyclopropyl group, a cyclobutyl group, and the like. The alkyl group of the above-mentioned haloalkyl group includes, but is not limited to, a straight-chain alkyl group, a branched-chain alkyl group, or a cycloalkyl group, such as a methyl group, an ethyl group, a propyl group, an isopropyl group, an n-butyl group, an isobutyl group, a cyclopropyl group, and a cyclobutyl group. The halogen atom may be a fluorine atom, a chlorine atom or a bromine atom, and the halogen atom substitutes any one or more hydrogen atoms of the alkyl group. The above-mentioned alkoxy group includes, but is not limited to, a cyclopropanediyl group, an oxetanediyl group, and the like. The halogen atom of the haloalkoxy group may be a fluorine, chlorine or bromine atom, and the halogen atom substitutes any one or more hydrogen atoms of the alkoxy group. The alkenyl group includes, but is not limited to, - CH=CH₂, -CH=CH₂CH₃, -CH₂CH=CH₂, and -CH₂CH=CH₂CH₃. The ester group includes, but is not limited to, methyl formate, ethyl formate, ethyl acetate, methyl propionate, ethyl propionate, propyl propionate, and the like.

In some embodiments, the cyclic sulfate compound has a structure represented by general formula (I-1), 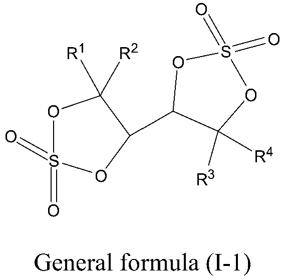
wherein R¹, R², R³ and R⁴ are each independently selected from any one of a group having a structure represented by general formula (II-1), a hydrogen atom, a halogen atom, a C1-C6 alkyl group, a C1-C6 haloalkyl group, a C1-C6 alkoxy group, a C1-C6 haloalkoxy group, a C2-C6 alkenyl group, a C2-C6 ester group, a cyano group, and a sulfonic acid group;
the general formula (II-1) is 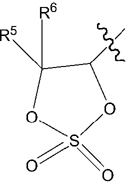
wherein R⁵ and R⁶ are each independently selected from any one of a group having a structure represented by the general formula (II-1), a hydrogen atom, a halogen atom, a C1-C6 alkyl group, a C1-C6 haloalkyl group, a C1-C6 alkoxy group, a C1-C6 haloalkoxy group, a C2-C6 alkenyl group, a C2-C6 ester group, a cyano group, and a sulfonic acid group.

Cyclic sulfate rings in the general formula (I-1) are all five-membered rings, and which may form a more compact SEI film. Compared with a six-membered ring, the five-membered ring has a greater ring tension and easily forms a film on the positive and negative electrodes. The six-membered ring has a smaller ring tension, and higher stability, and slowly forms a film on the negative electrode. Therefore, the efficiency of generating an SEI film that blocks electrons is low, which affects the effect of the SEI film.

In some embodiments, R¹, R², R³, R⁴, R⁵ and R⁶ mentioned above are each independently selected from any one of a group having a structure represented by the general formula (II-1), a hydrogen atom, a halogen atom, a C1-C3 alkyl group, a C1-C3 haloalkyl group, a C1-C3 alkoxy group, a C1-C3 haloalkoxy group, a C2-C3 alkenyl group, a C1-C3 ester group, a cyano group, and a sulfonic acid group.

In some embodiments, R¹, R², R³, R⁴, R⁵ and R⁶ mentioned above are each independently selected from any one of a group having a structure represented by the general formula (II-1), a hydrogen atom, a halogen atom, a C1-C3 alkyl group, and a C1-C3 haloalkyl group.

In some embodiments, R¹, R², R³, R⁴, R⁵ and R⁶ mentioned above are each independently selected from any one of a group having a structure represented by the general formula (II-1), a hydrogen atom, an F atom, a Cl atom, a Br atom, a methyl group, an ethyl group, a propyl group, and an isopropyl group.

In some embodiments, the group mentioned above having the structure represented by general formula (II-1) is selected from any one of the following groups: 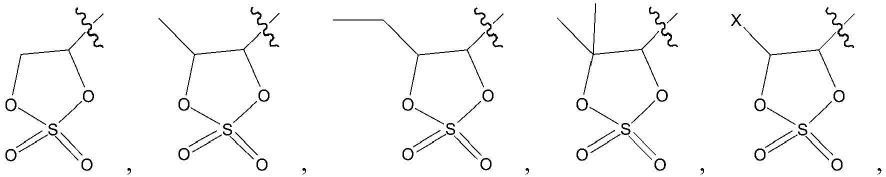 and 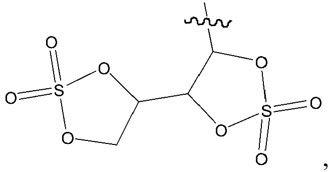 wherein X is an F atom, a Cl atom, or a Br atom.

In some embodiments, R¹, R², R³ and R⁴ mentioned above are each independently selected from 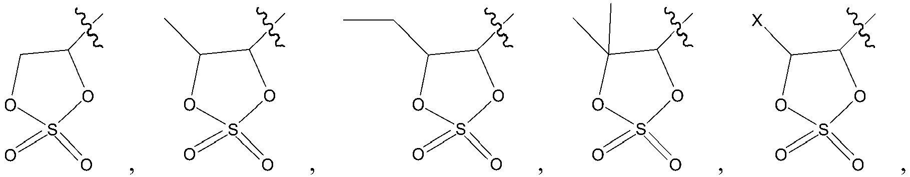 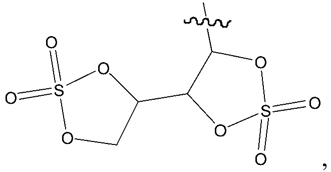 a hydrogen atom, an F atom, a Cl atom, a Br atom, a methyl group, an ethyl group, a propyl group, and an isopropyl group, and X is an F atom.

In some embodiments, R¹, R², R³ and R⁴ mentioned above are each independently selected from 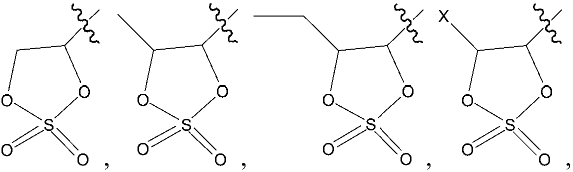 a hydrogen atom, a methyl group, and an ethyl group, and X is an F atom.

In some embodiments, the cyclic sulfate compound mentioned above is selected from one or more of the following compounds:

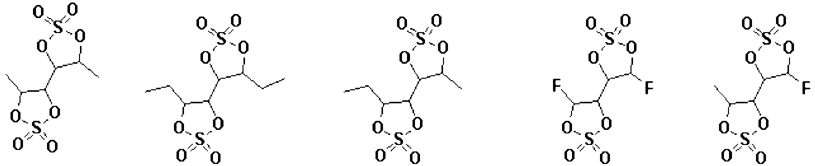

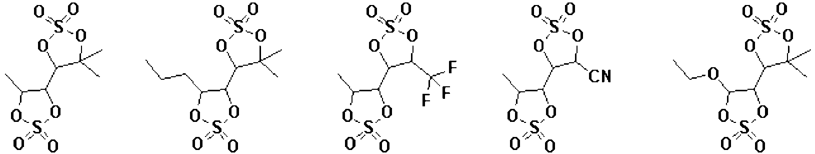

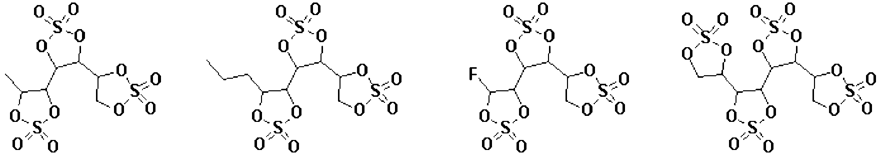

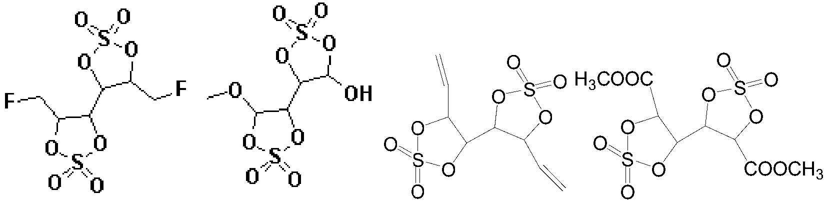

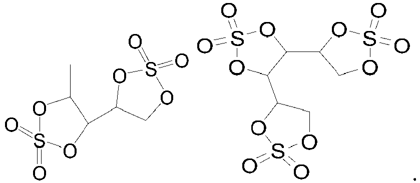

Methods of preparing some of the above cyclic sulfate compounds are simpler, easier to industrially promote and implement, and have a more stable effect for improving the life of the secondary battery.

In some embodiments, the 5-12 membered aromatic heterocyclic organic bases include one or more selected from the group consisting of a compound having a structure represented by general formula (III), a compound having a structure represented by general formula (IV), and a compound having a structure represented by general formula (V), where 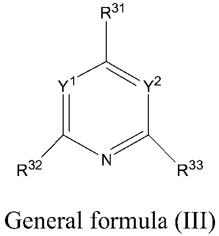 in the general formula (III), Y¹and Y²are each independently a C or N element; R³¹, R³²and R³³ are each independently selected from any one of a hydrogen atom, a halogen atom, a C1-C6 alkyl group, a C2-C6 alkenyl group, a C2-C6 alkynyl group, -R³⁴OH, -R³⁵N R³⁶R³⁷ and-R³⁸-O-R³⁹; R³⁴, R³⁵ and R³⁸ are each independently selected from any one of a C0-C6 alkylene group and a C2-C6 alkenylene group; R³⁶, R³⁷ and R³⁹ are each independently selected from any one of a hydrogen atom, a halogen atom, a C1-C6 alkyl group and a C1-C6 haloalkyl group; any carbon atom in the C1-C6 alkyl group as the R³⁶, R³⁷ and R³⁹ is optionally substituted with a heteroatom; the heteroatom is an N atom, an S atom or a P atom; and optionally, the R³⁶ and R³⁷ are connected to form a ring; 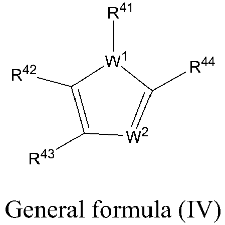 in the general formula (IV), W¹ is C, N, O or S; W² is C or N; at least one of W¹ and W² is N; R⁴¹, R⁴², R⁴³ and R⁴⁴ are each independently selected from any one of a hydrogen atom, a halogen atom, a C1-C6 alkyl group, a C2-C6 alkenyl group, a C2-C6 alkynyl group, -R⁴⁵OH, - R⁴⁶N R⁴⁷R⁴⁸ and -R⁴⁹-O-R⁵⁰; R⁴⁵, R⁴⁶ and R⁴⁹ are each independently selected from any one of a C0-C6 alkylene group and a C2-C6 alkenylene group; and R⁴⁷, R⁴⁸ and R⁵⁰ are each independently selected from any one of a hydrogen atom, a halogen atom, a C1-C6 alkyl group and a C1-C6 haloalkyl group; and 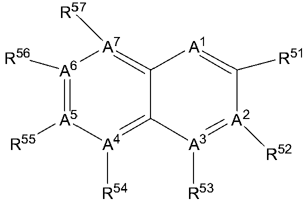 in the general formula (V), A¹, A², A³, A⁴, A⁵, A⁶ and A⁷ are each independently C or N; R⁵¹, R⁵², R⁵³, R⁵⁴, R⁵⁵, R⁵⁶ and R⁵⁷ are any one of a hydrogen atom, a C1-C6 alkyl group, a C2-C6 alkenyl group, a C2-C6 alkynyl group, -OH, -R⁵⁸N R⁵⁹R⁶⁰ and an alkoxy group; R⁵⁸is selected from any one of a C0-C6 alkylene group and a C2-C6 alkenylene group; and R⁵⁹ and R⁶⁰ are each independently selected from any one of a hydrogen atom, a halogen atom, a C1-C6 alkyl group and a C1-C6 haloalkyl group.

Each of the compounds of the above general formulas has good dispersibility and stability in the electrolyte, and is effective in absorbing acid, so that long-term mitigation of damage to the positive and negative electrode interfaces by the acid in the electrolyte can be achieved, and the battery cycling performance can be further improved.

In some embodiments, in general formula (III), R³¹, R³² and R³³ are each independently selected from any one of a hydrogen atom, a halogen atom, a C1-C4 alkyl group, a C3-C5 alkenyl group, a C3-C5 alkynyl group, -R³⁴OH, -R³⁵N R³⁶R³⁷ and -R³⁸-O-R³⁹, R³⁴, R³⁵ and R³⁸are each independently selected from any one of C0-C3 alkylene groups, R³⁶, R³⁷ and R³⁹ are each independently selected from any one of a hydrogen atom, a halogen atom, a C1-C3 alkyl group and a C1-C3 haloalkyl group, any carbon atom in the C1-C6 alkyl group as the R³⁶, R³⁷ and R³⁹ is optionally substituted with a heteroatom, the heteroatom is an N atom, and optionally, the R³⁶ and R³⁷ are connected to form a 5-membered or 6-membered aliphatic heterocycle.

In some embodiments, R³¹, R³² and R³³ are each independently selected from any one of a hydrogen atom, a halogen atom, a methyl group, an ethyl group, an n-propyl group, an isopropyl group, an allyl group, a propargyl group, -OH, -CH₃OH, -NH₂, -CH₂NH₂, -N(CH₃)₂, O-CH₃ and 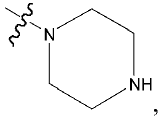 and optionally, the R³¹, R³² and R³³ are each independently selected from any one of a hydrogen atom, an F atom, a methyl group, an ethyl group, an n-propyl group, an isopropyl group, -CH₃OH, -CH₂NH₂, -N(CH₃)₂, O-CH₃ and

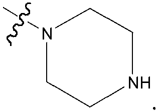

The more nitrogen atoms there are in the compound of the structure represented in the general formula (III), the better the absorption of the acid, but the more nitrogen atoms there are, the more reactive it is, the more likely it is to be oxidized by oxides of the positive electrode, which affects the absorption of the acid. In some embodiments, the compound having the structure represented by the general formula (III) is selected from any one or more of the following compounds: 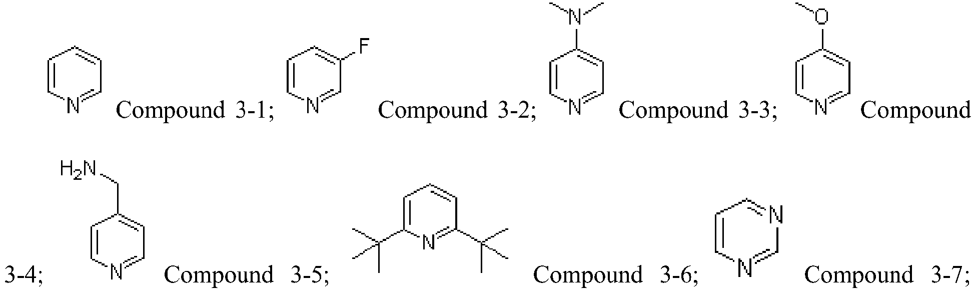 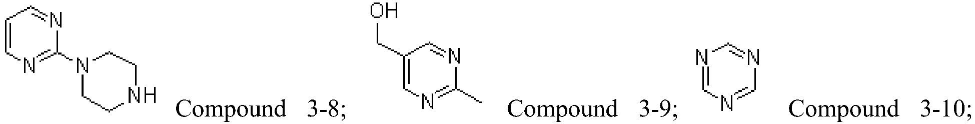 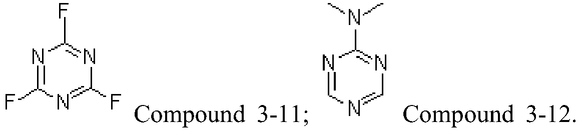 The acid absorption performance and antioxidant performance of the above compounds are relatively ideal, so the effect of improving the cycling performance of the battery is more obvious.

In some embodiments, in the general (IV), W¹ is N and W² is C or N.

In some embodiments, R⁴¹, R⁴², R⁴³ and R⁴⁴ are each independently selected from any one of a hydrogen atom, a halogen atom, a C1-C4 alkyl group, a C3-C5 alkenyl group, a C3-C5 alkynyl group, -R⁴⁴OH, -R⁴⁵N R⁴⁶R⁴⁷, and -R⁴⁸-O-R⁴⁹, R⁴⁴, R⁴⁵ and R⁴⁸ are each independently selected from any one of C0-C4 alkylene groups, and R⁴⁶, R⁴⁷ and R⁴⁹ are each independently selected from any one of a hydrogen atom, a halogen atom, a C1-C4 alkyl group and a C1-C4 haloalkyl group.

In some embodiments, R⁴¹, R⁴², R⁴³ and R⁴⁴ are each independently selected from any one of a hydrogen atom, an F atom, a methyl group, an ethyl group, a propyl group, a cyclopropyl group, an allyl group, a propargyl group, -OH, -CH₃OH, -NH₂, -NHCH₃, -CH₂NH₂, -N(CH₃)₂ and O-CH₃.

In some embodiments, R⁴¹, R⁴², R⁴³ and R⁴⁴ are each independently selected from any one of a hydrogen atom, an F atom, a methyl group, a propyl group, a cyclopropyl group, an allyl group, -CH₃OH, -NH₂, -NHCH₃ and -N(CH₃)₂.

The more nitrogen atoms there are in the compound of the structure represented in the general formula (IV), the better the absorption of the acid, but the more nitrogen atoms there are, the more reactive it is, the more likely it is to be oxidized by oxides of the positive electrode, which affects the absorption of the acid. In some embodiments, the compound having the structure represented by the general formula (IV) is selected from any one or more of the following compounds: 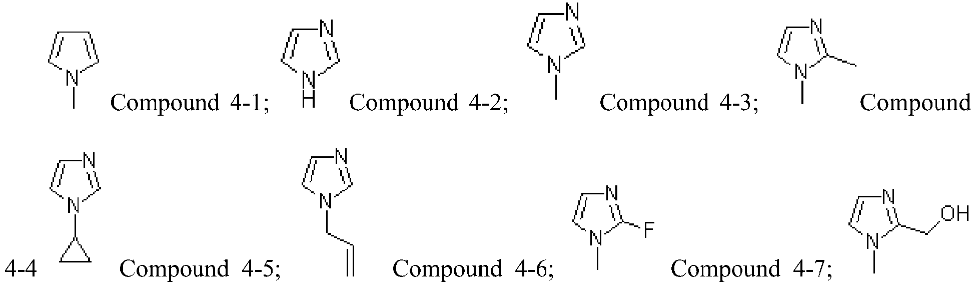 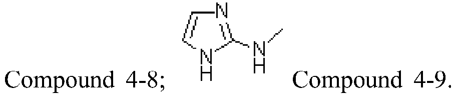 The acid absorption performance and antioxidant performance of the above compounds are relatively ideal, so the effect of improving the cycling performance of the battery is more obvious.

In some embodiments, in the general formula (V), A¹ is N, and A², A³, A⁴, A³, A⁶ and A⁷ are each independently C or N.

In some embodiments, R⁵¹, R⁵², R⁵³, R⁵⁴, R⁵⁵, R⁵⁶ and R⁵⁷ are any one of a hydrogen atom, a C1-C4 alkyl group, a C2-C4 alkenyl group, a C2-C4 alkynyl group, -OH, -R⁵⁸N R⁵⁹R⁶⁰ and an alkoxy group, R⁵⁸ is selected from any one of C0-C3 alkylene groups, and R⁵⁹ and R⁶⁰ are each independently selected from any one of a hydrogen atom, a halogen atom, a C1-C3 alkyl group and a C1-C3 haloalkyl group.

In some embodiments, R⁵¹, R⁵², R⁵³, R⁵⁴, R⁵⁵, R⁵⁶ and R⁵⁷ are any one of a hydrogen atom, a methyl group, an ethyl group, an allyl group, a propargyl group, -OH, -NH₂, -CH₂NH₂, -N(CH₃)₂ and O-CH₃.

In some embodiments, R⁵¹, R⁵², R⁵³, R⁵⁴, R⁵⁵, R⁵⁶ and R⁵⁷ are any one of a hydrogen atom, a methyl group, an ethyl group, and O-CH₃.

The more nitrogen atoms there are in the compound of the structure represented in the general formula (V), the better the absorption of the acid, but the more nitrogen atoms there are, the more reactive it is, the more likely it is to be oxidized by oxides of the positive electrode, which affects the absorption of the acid. In some embodiments, the compound having the structure represented by the general formula (V) is selected from any one or more of the following compounds: 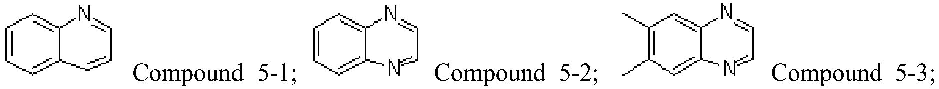 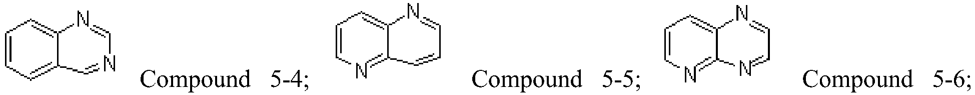 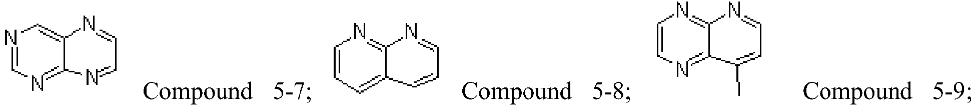 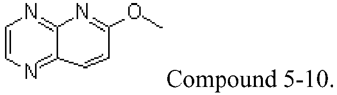 The acid absorption performance and antioxidant performance of the above compounds are relatively ideal, so the effect of improving the cycling performance of the battery is more obvious.

In some embodiments, the 5-12 membered aliphatic heterocyclic organic bases include one or more selected from the group consisting of a compound having a structure represented by general formula (VI) and a compound having a structure represented by general formula (VII), where 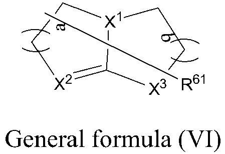 in the general formula (VI), X¹, X² and X³ are each independently C or N and at least one of them is required to be N; a and b are each independently an integer of 0-3; each R⁶¹ is independently selected from any one of a hydrogen atom, a halogen atom, a C1-C6 alkyl group, a C2-C6 alkenyl group, a C2-C6 alkynyl group, -R⁶²OH, -R⁶³N R⁶⁴R⁶⁵ and -R⁶⁶-O-R⁶⁷; R⁶², R⁶³ and R⁶⁴ are each independently selected from any one of a C0-C6 alkylene group and a C2-C6 alkenylene group; and R⁶⁵, R⁶⁶ and R⁶⁷ are each independently selected from any one of a hydrogen atom, a halogen atom, a C1-C6 alkyl group and a C1-C6 haloalkyl group, and 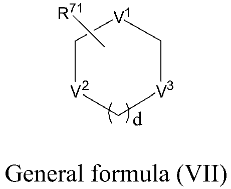 in the general formula (VI), V¹, V² and V³ are each independently C or N and at least one of them is required to be N; d is an integer of 0-3; each R⁷¹ is independently selected from any one of a hydrogen atom, a halogen atom, a C1-C6 alkyl group, a C2-C6 alkenyl group, a C2-C6 alkynyl group, -R⁷²OH, -R⁷³N R⁷⁴R⁷⁵ and -R⁷⁶-O-R⁷⁷; R⁷², R⁷³ and R⁷⁴ are each independently selected from any one of a C0-C6 alkylene group and a C2-C6 alkenylene group; and R⁷⁵, R⁷⁶ and R⁷⁷ are each independently selected from any one of a hydrogen atom, a halogen atom, a C1-C6 alkyl group and a C1-C6 haloalkyl group.

In some embodiments, in the general formula (VI), X¹ is N, and X² and X³ are each independently C or N.

In some embodiments, a and b are each independently 0, 1 or 2.

In some embodiments, each R⁶¹ is independently selected from any one of a hydrogen atom, a halogen atom, a C1-C4 alkyl group, a C3-C5 alkenyl group, a C3-C5 alkynyl group, -R⁶²OH, - R⁶³N R⁶⁴R⁶⁵, and -R⁶⁶-O-R⁶⁷, R⁶², R⁶³ and R⁶⁴ are each independently selected from any one of C0-C3 alkylene groups, and R⁶⁵, R⁶⁶ and R⁶⁷ are each independently selected from any one of a hydrogen atom, a halogen atom, a C1-C4 alkyl group and a C1-C4 haloalkyl group.

In some embodiments, each R⁶¹ is independently selected from any one of a hydrogen atom, an F atom, a methyl group, an ethyl group, a hydroxyl group, -NH₂, and -N(CH₃)₂.

The more nitrogen atoms there are in the compound of the structure represented in the general formula (IV), the better the absorption of the acid, but the more nitrogen atoms there are, the more reactive it is, the more likely it is to be oxidized by oxides of the positive electrode, which affects the absorption of the acid. In some embodiments, the compound having the structure represented by the general formula (VI) is selected from any one or more of the following compounds: 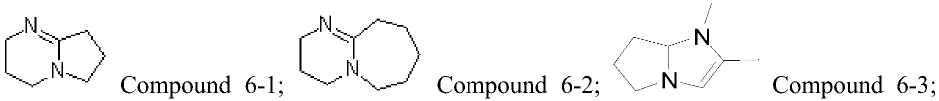 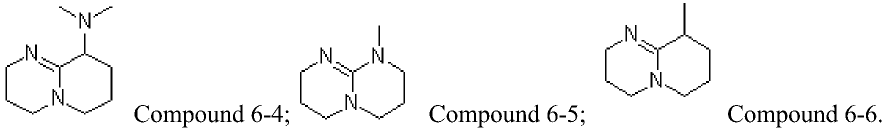 The acid absorption performance and antioxidant performance of the above compounds are relatively ideal, so the effect of improving the cycling performance of the battery is more obvious.

In some embodiments, in the general (VII), d is 0 or 1.

In some embodiments, each R⁷¹ is independently selected from any one of a hydrogen atom, a halogen atom, a C1-C4 alkyl group, a C3-C5 alkenyl group, a C3-C5 alkynyl group, -R⁷²OH, - R⁷³N R⁷⁴R⁷⁵, and -R⁷⁶-O-R⁷⁷, R⁷², R⁷³ and R⁷⁴ are each independently selected from any one of C0-C3 alkylene groups, and R⁷⁵, R⁷⁶ and R⁷⁷ are each independently selected from any one of a hydrogen atom, a halogen atom, a C1-C4 alkyl group and a C1-C4 haloalkyl group.

In some embodiments, each R⁷¹ is independently selected from any one of a hydrogen atom, an F atom, a methyl group, an ethyl group, an n-propyl group, an isopropyl group, a hydroxyl group, -NH₂, and -N(CH₃)₂.

In some embodiments, each R⁷¹ is independently selected from any one of a hydrogen atom, an F atom, a methyl group, an ethyl group, an n-propyl group, and an isopropyl group.

The more nitrogen atoms there are in the compound of the structure represented in the general formula (VII), the better the absorption of the acid, but the more nitrogen atoms there are, the more reactive it is, the more likely it is to be oxidized by oxides of the positive electrode, which affects the absorption of the acid. In some embodiments, the compound having the structure represented by the general formula (VII) is selected from any one or more of the following compounds: 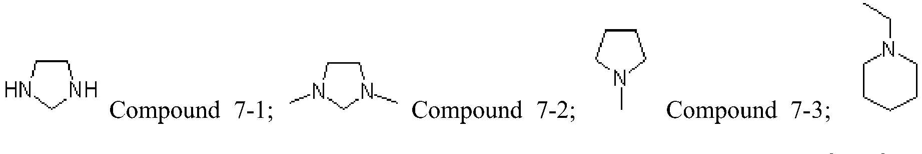 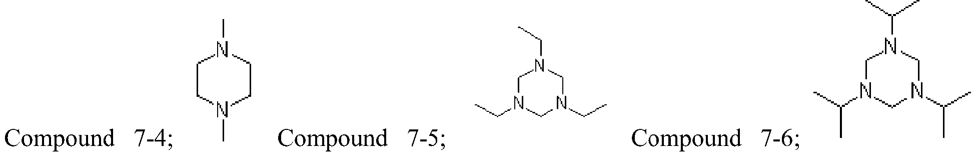 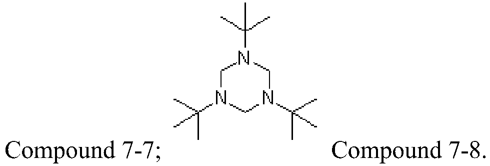 The acid absorption performance and antioxidant performance of the above compounds are relatively ideal, so the effect of improving the cycling performance of the battery is more obvious.

For the amount of the cyclic sulfate compound in the above embodiments of the present application, reference may be made to the amount of a conventional cyclic sulfate compound in a conventional non-aqueous electrolyte. In some embodiments, mass percent of the first additive in the non-aqueous electrolyte is W1, W1 is optionally 0.001-20%, e.g. 0.001%, 0.005%, 0.01%, 0.05%, 0.1%, 0.5%, 1%, 3%, 5%, 10%, 15%, or 20%, and W1 is further optionally between 0.1% and 5%. By utilizing the cyclic sulfate compound to form a fully organic and inorganic mixed SEI film with more stability and stronger electron blocking capability, not only the cycling performance of a secondary battery may be effectively improved, but also the output power of the secondary battery may be improved. Under the limitation on the above mass content, it is avoided that the SEI film cannot fully function due to too little content of the cyclic sulfate compound, and that too much cyclic sulfate compound leads to excessive viscosity of the electrolyte and too thick SEI film formed at the negative electrode, deteriorating the electrical conductivity of the electrolyte and further deteriorating the improvement in the cycling performance and charging capacity.

For the amount of the organic base additives in each of the above embodiments of the present application, reference may be made to the amount of conventional organic base additives in a conventional non-aqueous electrolyte. In some embodiments, mass percent of the second additive in the non-aqueous electrolyte is W2, W2 is optionally 0.001-20%, e.g. 0.001%, 0.005%, 0.01%, 0.05%, 0.1%, 0.5%, 1%, 3%, 5%, 10%, 15%, or 20%, and W2 is further optionally between 0.1% and 5%. The amount of the second additive is adjusted within the above mass percent range to realize flexible adjustment of acid in the electrolyte.

In some embodiments, 0.01≤W1/W2≤10, preferably 0.05≤W1/W2≤5. By adjusting the ratio of the two additives, utilizing the first additive to form a sufficient SEI film on the basis of not affecting conductivity of the electrolyte, while utilizing the second additive to further fully absorb the acid in the electrolyte, the two additives are fully used, effectively enhancing the cycling performance and storage performance of the battery.

In some embodiments, the non-aqueous electrolyte further includes an electrolyte. Any electrolyte that may be commonly used in a non-aqueous electrolyte may be considered for application in the non-aqueous electrolyte of the present application. Those skilled in the art may make a selection according to a battery system to which the non-aqueous electrolyte is applied, e.g. to select a conventional electrolyte suitable for the lithium-ion secondary battery or sodium-ion secondary battery. In some embodiments, the electrolyte optionally includes an alkali metal salt electrolyte; the electrolyte optionally includes a lithium salt or a sodium salt; the lithium salt optionally includes one or more selected from the group consisting of lithium hexafluorophosphate, lithium perchlorate, lithium hexafluoroarsenate, lithium bis(fluorosulfonyl)imide, and lithium bis(trifluoromethanesulfonyl)imide; the sodium salt includes one or more selected from the group consisting of sodium hexafluorophosphate, sodium difluoro(oxalato)borate, sodium perchlorate, sodium bis(fluorosulfonyl)imide, sodium bis(trifluoromethanesulfonyl)imide, and sodium trifluoromethanesulfonate.

For the content of electrolyte in the non-aqueous electrolyte, reference may be made to an electrolyte content in a conventional non-aqueous electrolyte. In some embodiments, the content of electrolyte in the non-aqueous electrolyte is 0.1-5 mol/L, e.g. may be 0.1 mol/L, 0.3 mol/L, 0.5 mol/L, 1 mol/L, 1.5 mol/L, 2 mol/L, 2.5 mol/L, 3 mol/L, 4 mol/L, or 5 mol/L, optionally 0.5-1.5 mol/L, and further optionally 0.7-1.2 mol/L.

In some embodiments, the non-aqueous electrolyte above further includes a non-aqueous solvent, the non-aqueous solvent optionally includes any one or more selected from the group consisting of cyclic carbonate, chain carbonate, a nitrile solvent, a ketone solvent, and a sulfone solvent, and the non-aqueous solvent further optionally includes one or more selected from the group consisting of ethylene carbonate, propylene carbonate, methyl ethyl carbonate, diethyl carbonate, dimethyl carbonate, dipropyl carbonate, methyl propyl carbonate, ethyl propyl carbonate, butylene carbonate, fluoroethylene carbonate, methyl formate, methyl acetate, ethyl acetate, propyl acetate, methyl propionate, ethyl propionate, propyl propionate, methyl butyrate, ethyl butyrate, 1,4-butyrolactone, sulfolane, dimethyl sulfone, methyl ethyl sulfone, diethyl sulfone, tetrahydrofuran, glycol dimethyl ether, dioxolane, acetone, acetonitrile, and butyronitrile. The above-mentioned non-aqueous solvents may be used alone or in combination of two or more. For example, in order to improve the load characteristic and low-temperature characteristic of the secondary battery, a mixed solvent of cyclic carbonate and chain carbonate may be used. When the non-aqueous electrolyte of the present application is applied to a solid battery, a solid solvent such as dimethyl sulfone may be used.

In addition to the above additives, the additives may further include a negative electrode film-forming additive or a positive electrode film-forming additive, or may further include an additive that can improve some performance of the battery, such as an additive that improves overcharge performance of the battery or an additive that improves high-temperature or low-temperature performance of the battery. In some embodiments of the present application, the above additives further include, but are not limited to, one or more selected from the group consisting of a sulfate compound, a sulfite compound, a sultone compound, a disulfonic acid compound, a nitrile compound, an aromatic compound, an isocyanate compound, a phosphazene compound, a cyclic anhydride compound, a phosphite compound, a phosphate compound, a borate compound, and a carboxylate compound.

### [Method of preparing a cyclic sulfate compound having a structure represented by general formula (I)]

The method for preparing the cyclic sulfate compound having the structure represented by the general formula (I) in the present application is referred to the following synthetic route:

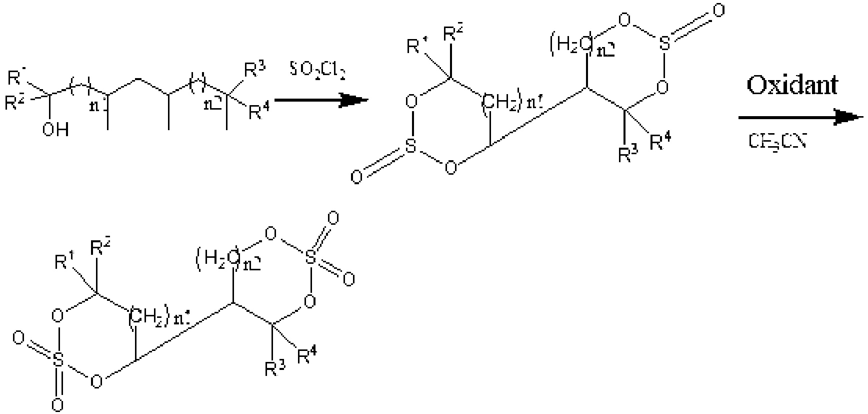

The reaction temperature of a first step is controlled to 30-60°C; a reaction temperature of a second step is controlled to 10-30°C. In the second step, a catalyst such as ruthenium(III) chloride hydrate is used for catalysis, and an oxidant may be sodium hypochlorite, ozone, and the like.

### [Positive electrode plate]

The positive electrode plate typically includes a positive electrode current collector and a positive electrode film layer provided on at least one surface of the positive electrode current collector, wherein the positive electrode film layer includes a positive electrode active material.

As an example, the positive electrode current collector has two opposite surfaces in its own thickness direction, and the positive electrode film layer is arranged on either or both of the opposite surfaces of the positive electrode current collector.

In some embodiments, the positive electrode current collector may be a metal foil or a composite current collector. For example, an aluminum foil may be used as the metal foil. The composite current collector may include a polymer material substrate layer and a metal layer formed on at least one surface of the polymer material substrate layer. The composite current collector may be formed by forming a metal material (such as aluminum, aluminum alloy, nickel, nickel alloy, titanium, titanium alloy, silver, and silver alloy) on a high molecular material substrate (such as a substrate of polypropylene (PP), polyethylene terephthalate (PET), polybutylene terephthalate (PBT), polystyrene (PS), or polyethylene (PE)).

In some embodiments, the positive electrode active material may be a positive electrode active material for batteries well known in the art. As an example, the positive electrode active material may include at least one of the following materials: a lithium-containing phosphate of olivine structure, a lithium transition metal oxide, and their respective modified compounds thereof. However, the present application is not limited to these materials, and other conventional materials that can be used as positive electrode active materials for batteries may also be used. Only a single one of or a combination of two or more of these positive electrode active materials can be used. Examples of lithium transition metal oxides may include, but are not limited to, at least one of a lithium-cobalt oxide (such as LiCoO₂), a lithium-nickel oxide (such as LiNiO₂), a lithium-manganese oxide (such as LiMnO₂ or LiMn₂O₄), a lithium-nickel-cobalt oxide, a lithium-manganese-cobalt oxide, a lithium-nickel-manganese oxide, a lithium-nickel-cobalt-manganese oxide (such as LiNi_{1/3}Co_{1/3}Mn_{1/3}O₂ (also abbreviated as NCM₃₃₃), LiNi_{0.5}Co_{0.2}Mn_{0.3}O₂ (also abbreviated as NCM₅₂₃), LiNi_{0.5}Co_{0.25}Mn_{0.25}O₂ (also abbreviated as NCM₂₁₁), LiNi_{0.6}Co_{0.2}Mn_{0.2}O₂ (also abbreviated as NCM₆₂₂), LiNi_{0.8}Co_{0.1}Mn_{0.1}O₂ (also abbreviated as NCM₈₁₁)), a lithium-nickel-cobalt-aluminum oxide (such as LiNi_{0.85}Co_{0.15}Al_{0.05}O₂), and a modified compound thereof. Examples of lithium-containing phosphates of olivine structure may include, but are not limited to, at least one of lithium iron phosphate (e.g., LiFePO₄ (also abbreviated as LFP)), lithium iron phosphate and carbon composites, lithium manganese phosphate (e.g., LiMnPO₄), lithium manganese phosphate and carbon composites, lithium iron manganese phosphate, and lithium iron manganese phosphate and carbon composites.

In some embodiments, the positive electrode film layer further optionally includes a binder. As an example, the binder may include at least one of polyvinylidene fluoride (PVDF), polytetrafluoroethylene (PTFE), vinylidene fluoride-tetrafluoroethylene-propylene terpolymer, vinylidene fluoride-hexafluoropropylene-tetrafluoroethylene terpolymer, tetrafluoroethylene-hexafluoropropylene copolymer, and fluoroacrylate resin.

In some embodiments, the positive electrode film layer further optionally includes a conductive agent. As an example, the conductive agent may include at least one of superconducting carbon, acetylene black, carbon black, Ketjen black, carbon dots, carbon nanotubes, graphene, and carbon nanofibers.

In some embodiments, the positive electrode plate may be prepared by: dispersing the above components, such as the positive electrode active material, the conductive agent, the binder, and any other component, for preparing the positive electrode plate in a solvent (e.g., N-methyl pyrrolidone) to form a positive electrode slurry; and applying the positive electrode slurry on the positive electrode current collector, drying, and cold pressing, to obtain the positive electrode plate.

### [Negative electrode plate]

The negative electrode plate includes a negative electrode current collector and a negative electrode film layer arranged on at least one surface of the negative electrode current collector, and the negative electrode film layer includes a negative electrode active material.

As an example, the negative electrode current collector has two surfaces opposite in its own thickness direction, and the negative electrode film layer is arranged on either one or both of the two opposite surfaces of the negative electrode current collector.

In some embodiments, the negative electrode current collector may be a metal foil or a composite current collector. For example, a copper foil may be used as the metal foil. The composite current collector may include a high molecular material substrate layer and a metal layer formed on at least one surface of the high molecular material substrate. The composite current collector can be formed by forming a metal material (copper, copper alloy, nickel, nickel alloy, titanium, titanium alloy, silver, and silver alloy, etc.) on a polymer material substrate (e.g., polypropylene (PP), polyethylene terephthalate (PET), polybutylene terephthalate (PBT), polystyrene (PS), polyethylene (PE), etc.).

In some embodiments, a negative electrode active material for the battery well known in the art may be used as the negative electrode active material. As an example, the negative electrode active material may include at least one of the following materials: artificial graphite, natural graphite, soft carbon, hard carbon, silicon-based material, tin-based material, lithium titanate, etc. The silicon-based material may be selected from at least one of elemental silicon, silicon-oxygen compound, silicon-carbon complex, silicon-nitrogen complex, and silicon alloy. The tin-based material may be selected from at least one of elemental tin, tin-oxygen compound, and tin alloy. However, the present application is not limited to these materials, and other conventional materials useful as negative electrode active materials for batteries can also be used. These negative electrode active materials may be used alone or in combination of two or more thereof.

In some embodiments, the negative electrode film layer has a porosity of 30-45%, optionally 37-42%. When the porosity of the negative electrode film layer on the negative electrode plate is less than 30%, there are few gaps between particles inside the negative electrode film layer, and the particle structure is damaged under extrusion, which increases the difficulty of electrolyte infiltration, increases the polarization of a battery cell, and deteriorates long-term cycling performance of the battery cell; whereas when the porosity of the negative electrode film layer on the negative electrode plate is greater than 45%, the plate rebounds significantly, resulting in a decrease in the compaction in actual use of the plate, and affecting the energy density of the battery cell.

The porosity of the negative electrode active material coating on the negative electrode plate was tested using an AccuPyc II 1340 true density instrument according to the instrument manual. The porosity of the negative electrode active material coating on the plate may be controlled by regulating a particle size of the negative electrode active material and a pressure in the cold pressing procedure.

In some embodiments, the negative electrode material layer includes a negative electrode active material; optionally, the negative electrode active material has D_{V}50≥6 µm; further optionally, the negative electrode active material has D_{V}50 between 15 µm and 20 µm. Since the first additive and the second additive form a SEI film with a certain thickness on the surface of the negative electrode plate, increasing the volumetric particle size of the negative electrode active material may reduce the area of contact between the negative electrode active material and the electrolyte, and reduce the side reactions of the surface of the negative electrode, thus improving the cycling and storage performances of the battery cell.

In the present application, the volumetric mean particle size Dv50 of the negative electrode active material has a meaning well-known in the art, and may be assayed using instruments and methods known in the art. For example, it can be determined by using a laser particle size analyzer (e.g., Master Size 3000) with reference to the GB/T 19077-2016 Particle size analysis-Laser diffraction method.

In some embodiments, the negative electrode film layer further optionally includes a binder. As an example, the binder may be selected from at least one of styrene butadiene rubber (SBR), polyacrylic acid (PAA), sodium polyacrylate (PAAS), polyacrylamide (PAM), polyvinyl alcohol (PVA), sodium alginate (SA), polymethacrylic acid (PMAA) and carboxymethyl chitosan (CMCS).

In some embodiments, the negative electrode film layer further optionally includes a conductive agent. As an example, the conductive agent may be selected from at least one of superconducting carbon, acetylene black, carbon black, Ketjen black, carbon dot, carbon nanotube, graphene, and carbon nanofiber.

In some embodiments, the negative electrode film layer further optionally includes other adjuvants, for example, a thickener (such as sodium carboxymethyl cellulose (CMC-Na)).

In some embodiments, the negative electrode plate may be prepared by: dispersing the above components, such as the negative electrode active material, the conductive agent, the binder and any other component, for preparing the negative electrode plate in a solvent (such as deionized water) to form a negative electrode slurry; and coating the negative electrode slurry on the negative electrode current collector, and performing drying and cold pressing processes to obtain the negative electrode plate.

### [Separator]

In some embodiments, the secondary battery further includes a separator. The type of the separator is not particularly limited in the present application, and any well-known separator with a porous structure having good chemical stability and mechanical stability can be selected.

In some embodiments, the material of the separator may be selected from at least one of glass fiber, non-woven cloth, polyethylene, polypropylene, and polyvinylidene fluoride. The separator may be either a single-layer thin film or a multilayer composite thin film without special limitations. When the separator is a multilayer composite thin film, the materials of the layers can be the same or different without special limitations.

In some embodiments, the positive electrode plate, the negative electrode plate, and the separator can be made into an electrode assembly by a winding process or a lamination process.

In some embodiments, the secondary battery may include an outer package. The outer packaging can be used for encapsulating the above electrode assembly and electrolyte.

In some embodiments, the outer package of the secondary battery may be a hard case, such as a hard plastic case, an aluminum case, a steel case, and the like. The outer package of the secondary battery may also be a soft pack, such as a pouch. The material of the soft pack may be a plastic, and examples of the plastic may include polypropylene, polybutylene terephthalate, polybutylene succinate, and the like.

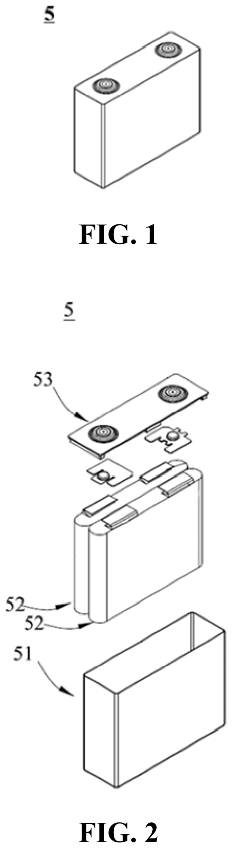

The shape of the secondary battery is not particularly limited in the present application, and may be a cylinder, a square, or any other shape. For example, Fig. 1 shows a secondary battery 5 with a square structure as an example.

In some embodiments, referring to Fig. 2, the outer package may include a case 51 and a cover plate 53. The case 51 may include a bottom plate and a side plate connected to the bottom plate, which enclose to form an accommodating cavity. The case 51 has an opening that communicates with the accommodating cavity, and the cover plate 53 may cover the opening to close the accommodating cavity. The positive electrode plate, the negative electrode plate, and the separator may be formed into an electrode assembly 52 by a winding process or a stacking process. The electrode assembly 52 is encapsulated within the accommodating cavity. The electrolyte infiltrates the electrode assembly 52. The number of electrode assemblies 52 included in the secondary battery 5 may be one or more, and may be selected by those skilled in the art according to specific actual requirements.

In some embodiments, the secondary batteries may be assembled into a battery module, the number of secondary batteries included in the battery module may be one or more, and the specific number may be selected by those skilled in the art according to the application and capacity of the battery module.

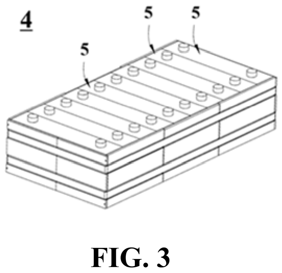

Fig. 3 shows a battery module 4 as an example. Referring to Fig. 3, in the battery module 4, a plurality of secondary batteries 5 can be sequentially arranged along the length direction of the battery module 4. Of course, any other arrangements are also possible. The plurality of secondary batteries 5 may further be fixed by fasteners.

Optionally, the battery module 4 may further include a shell having an accommodating space, in which the plurality of secondary batteries 5 are accommodated.

In some embodiments, the above battery modules may be further assembled into a battery pack, the number of battery modules included in the battery pack may be one or more, and the specific number may be selected by those skilled in the art based on the application and capacity of the battery pack.

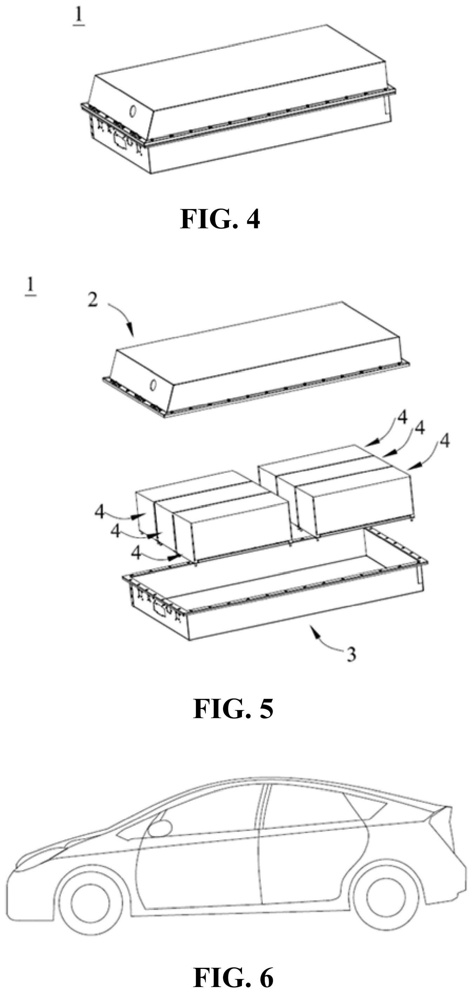

Figs. 4 and 5 show a battery pack 1 as an example. Referring to Figs. 4 and 5, the battery pack 1 may include a battery box and a plurality of battery modules 4 provided in the battery box. The battery box includes an upper box 2 and a lower box 3, wherein the upper box 2 can cover the lower box 3 and forms an enclosed space for accommodating the battery module 4. The plurality of battery modules 4 may be arranged in the battery box in any manner.

In addition, the present application further provides an electrical apparatus, including at least one of the secondary battery, the battery module, or the battery pack provided in the present application. The secondary battery, the battery module, or the battery pack can be used as a power source for the electrical apparatus, and can also be used as an energy storage unit for the electrical apparatus. The electrical apparatus may include, but is not limited to, a mobile device (such as a mobile phone, and a laptop, etc.), an electric vehicle (such as an all-electric vehicle, a hybrid electric vehicle, a plug-in hybrid electric vehicle, an electric bicycle, an electric scooter, an electric golf cart, and an electric truck, etc.), an electric train, a ship, a satellite, an energy storage system, etc.

For the electrical apparatus, the secondary battery, the battery module, or the battery pack may be selected according to its use requirements.

Fig. 6 is an example of an electrical apparatus. The electrical apparatus is an all-electric vehicle, a hybrid electric vehicle, a plug-in hybrid electric vehicle, or the like. In order to meet the requirements of the electrical apparatus for high power and high energy density of a secondary battery, a battery pack or a battery module may be used.

### Examples

Examples of the present application will be described below. The embodiments described below are illustrative and only used to explain the present application, and cannot be construed as limiting the present application. Where specific techniques or conditions are not specified in the examples, the techniques or conditions described in the literature of the art or the product specifications are followed. Where manufacturers are not specified, the reagents or instruments used are conventional products and are commercially available, and information of other reagents or compounds is recorded in Table 1.

**Table 1**

| Material | Structural formula | CAS number |
|---|---|---|
| 1,6-dideoxy-galactitol | 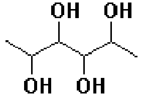 | 25289-20-7 |
| 3,4,5,6-octanetetrol | 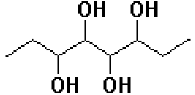 | 2165939-88-6 |
| 2,3,4,5-heptanetetrol | 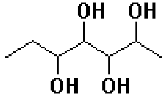 | 2629309-49-3 |
| 1,2,3,4,5.6-heptanehexol | 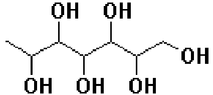 | 688007-16-1 |
| Octitol | 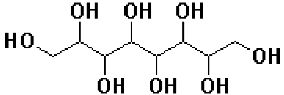 | 63976-32-9 |
| Compound 12 | 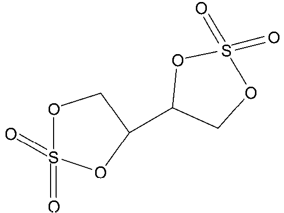 | 1431298-10-0 |
| Compound 13 | 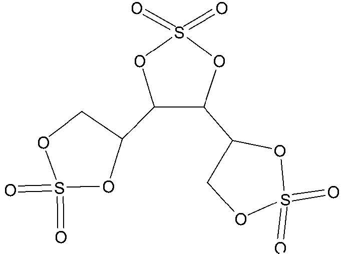 | 2793408-99-6 |

### Synthesis Example 1: Synthesis of Compound 1

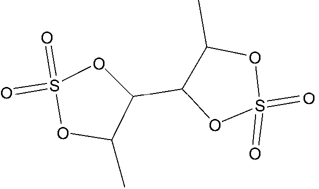

Step 1: Add 300 g (2 mol) of solid 1,6-dideoxy-galactitol into a 2L three-necked flask, start stirring, add 523 g (4.4 mol) of sulfoxide chloride dropwise into the three-necked flask, control the temperature at about 15°C during adding, hold the temperature for reaction for 4h at 45°C after dropwise addition, with a large amount of pasty solid precipitated from a reaction solution; after cooling, slowly add 1L of deionized water, stir to scatter the reaction system quickly, then beat and wash the solid obtained by filtering with deionized water several times until its pH is neutral, followed by drying a filter cake at 60°C under a reduced pressure to obtain an intermediate product.

Step 2: Add 184.2 g (0.8 mol) of the intermediate product 1 to a 3L three-necked flask, add 1000 mL of acetonitrile, add 80 mg of ruthenium(III) chloride hydrate catalyst, perform nitrogen replacement for the system, then cool the system to 20°C, start stirring, drop 2000 g of 20% sodium hypochlorite aqueous solution within 1 hour, and control the reaction temperature at 10-20°C; after dropwise addition, stir at 10-20°C for 10 min, perform liquid separation, and quench the organic phase with a sodium sulfite aqueous solution until the potassium iodide starch test paper does not turn blue; perform liquid separation again, and concentrate an organic layer to crystallize acetonitrile to obtain a white-powder solid, i.e., the compound 1 mentioned above. 1H-NMR, CD₃CN, δ ppm 5.42-5.39 (m, 2H), 5.36-5.34 (m, 2H), 1.67-1.65 (d, 6H).

### Synthesis Example 2: Synthesis of compound 2

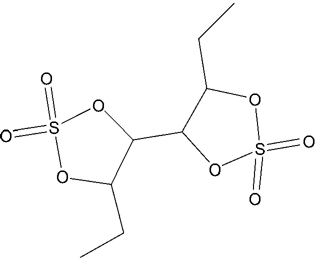

Step 1: Add 356.5 g (2 mol) of solid 3,4,5,6-octanetetrol into a 2L three-necked flask, start stirring, add 523 g (4.4 mol) of sulfoxide chloride dropwise into the three-necked flask, control the temperature at about 15°C during adding, hold the temperature for reaction for 4h at 45°C after dropwise addition, with a large amount of pasty solid precipitated from a reaction solution; after cooling, slowly add 1L of deionized water, stir to scatter the reaction system quickly, then beat and wash the solid obtained by filtering with deionized water several times until its pH is neutral, followed by drying a filter cake at 60°C under a reduced pressure to obtain an intermediate product.

Step 2: Add 216.2 g (0.8 mol) of the intermediate product 1 to a 3L three-necked flask, add 1000 mL of acetonitrile, add 80 mg of ruthenium(III) chloride hydrate catalyst, perform nitrogen replacement for the system, then cool the system to 20°C, start stirring, drop 2000 g of 20% sodium hypochlorite aqueous solution within 1 hour, and control the reaction temperature at 10-20°C; after dropwise addition, stir at 10-20°Cfor 10 min, perform liquid separation, and quench the organic phase with a sodium sulfite aqueous solution until the potassium iodide starch test paper does not turn blue; perform liquid separation again, and concentrate an organic layer to crystallize acetonitrile to obtain the compound 2.

### Synthesis Example 3: Synthesis of compound 3

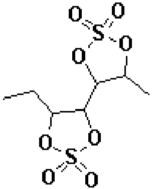

Step 1: Add 328.4 g (2 mol) of solid 2,3,4,5-heptanetetrol into a 2L three-necked flask, start stirring, add 523 g (4.4 mol) of sulfoxide chloride dropwise into the three-necked flask, control the temperature at about 15°C during adding, hold the temperature for reaction for 4h at 45°C after dropwise addition, with a large amount of pasty solid precipitated from a reaction solution; after cooling, slowly add 1L of deionized water, stir to scatter the reaction system quickly, then beat and wash the solid obtained by filtering with deionized water several times until its pH is neutral, followed by drying a filter cake at 60°C under a reduced pressure to obtain an intermediate product.

Step 2: Add 205 g (0.8 mol) of the intermediate product 1 to a 23 three-necked flask, add 1000 mL of acetonitrile with stirring until the solid was completely dissolved, add 80 mg of ruthenium(III) chloride hydrate catalyst, perform nitrogen replacement for the system, then cool the system to 20°C, start stirring, drop 2000 g of 20% sodium hypochlorite aqueous solution within 1 hour, and control the reaction temperature at 10-20°C; after dropwise addition, stir at 10-20°Cfor 10 min, perform liquid separation, and quench the organic phase with a sodium sulfite aqueous solution until the potassium iodide starch test paper does not turn blue; perform liquid separation again, and concentrate an organic layer to crystallize acetonitrile to obtain the compound 3 (163.1g, yield 82.8%).

### Synthesis Example 4: Synthesis of compound 4

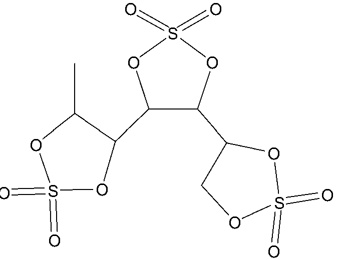

Step 1: Add 392.4 g (2 mol) of solid 1,2,3,4,5.6-heptanehexanol into a 2L three-necked flask, start stirring, add 784.5 g (6.6 mol) of sulfoxide chloride dropwise into the three-necked flask, control the temperature at about 15°C during adding, hold the temperature for reaction for 4h at 45°C after dropwise addition, with a large amount of pasty solid precipitated from a reaction solution; after cooling, slowly add 1L of deionized water, stir to scatter the reaction system quickly, then beat and wash the solid obtained by filtering with deionized water several times until its pH is neutral, followed by drying a filter cake at 60°C under a reduced pressure to obtain an intermediate product.

Step 2: Add 140 g (0.4 mol) of the intermediate product 1 to a 4L three-necked flask, add 1000 mL of acetonitrile, add 110 mg of ruthenium(III) chloride hydrate catalyst, perform nitrogen replacement for the system, then cool the system to 20°C, start stirring, drop 1500 g of 20% sodium hypochlorite aqueous solution within 1 hour, and control the reaction temperature at 10-20°C; after dropwise addition, stir at 10-20°Cfor 10 min, perform liquid separation, and quench the organic phase with a sodium sulfite aqueous solution until the potassium iodide starch test paper does not turn blue; perform liquid separation again, and concentrate an organic layer to crystallize acetonitrile to obtain the compound 4.

### Synthesis Example 5: Synthesis of compound 5

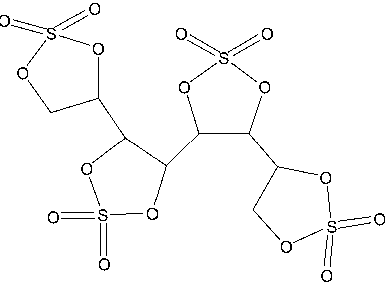

Step 1: Add 484 g (2 mol) of solid octitol into a 2L three-necked flask, start stirring, add 1046 g (8.8 mol) of sulfoxide chloride dropwise into the three-necked flask, control the temperature at about 15°C during adding, hold the temperature for reaction for 4h at 45°C after dropwise addition, with a large amount of pasty solid precipitated from a reaction solution; after cooling, slowly add 1L of deionized water, stir to scatter the reaction system quickly, then beat and wash the solid obtained by filtering with deionized water several times until its pH is neutral, followed by drying a filter cake at 60°C under a reduced pressure to obtain an intermediate product.

Step 2: Add 183.2 g (0.4 mol) of the intermediate product to a 4L three-necked flask, add 1000 mL of acetonitrile, add 150 mg of ruthenium(III) chloride hydrate catalyst, perform nitrogen replacement for the system, then cool the system to 20°C, start stirring, drop 2000 g of 20% sodium hypochlorite aqueous solution within 1 hour, and control the reaction temperature at 10-20°C; after dropwise addition, stir at 10-20°Cfor 10 min, perform liquid separation, and quench the organic phase with a sodium sulfite aqueous solution until the potassium iodide starch test paper does not turn blue; perform liquid separation again, and concentrate an organic layer to crystallize acetonitrile to obtain the compound 5.

In addition, compounds were synthesized by a method with reference to that in Synthesis Example 1, wherein the corresponding substrates in Table 2 were used to substitute 1,6- dideoxy-galactitol.

**Table 2**

| Compound | Chemical formula | Substrate | Compound LC-MS |
|---|---|---|---|
| 6 | 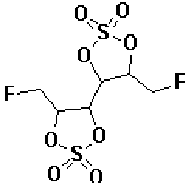 | 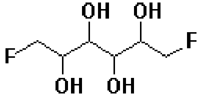 | 310.25 |
| | | CAS Number: 35827-51-1 | |
| 7 | 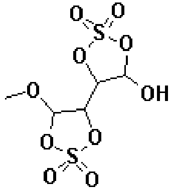 | 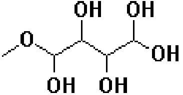 | 292.24 |
| | | CAS Number: 113421-87-7 | |
| 8 | 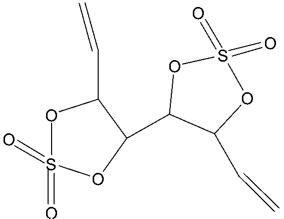 | 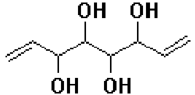 | 298.29 |
| | | CAS Number: 301523-44-4 | |
| 9 | 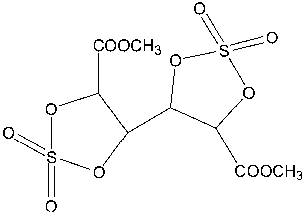 | 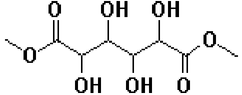 | 362.34 |
| | | CAS Number: 24808-45-5 | |
| 10 | 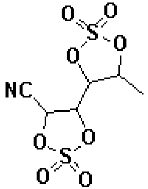 | 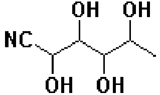 | 285.25 |
| | | CAS Number: 7460-93-7 | |
| 11 | 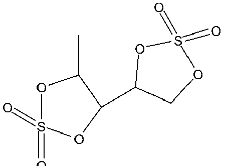 | 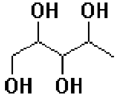 | 260.24 |
| | | CAS Number: 25289-19-4 | |

### Example 1

### Preparation of secondary battery:

Electrolyte composition: Compound 1 was used as a first additive, with its mass content in the electrolyte being 2%; Compounds 3-6 were used as second additives, with their mass contents in the electrolyte being 2%; lithium hexafluorophosphate LiPF₆ was used as an electrolyte, with its content in the electrolyte being 10%; a mixture of EC + EMC (ethylene carbonate + ethyl methyl carbonate) with a volume ratio of 3:7 was used as a solvent.

### Preparation of positive electrode plate:

A positive electrode active material lithium iron phosphate (LiFePO₄), a conductive agent acetylene black and a binder polyvinylidene fluoride (PVDF) were dissolved in a solvent N-methylpyrrolidone (NMP) according to a mass ratio of 90:5:5, and stirred and mixed sufficiently to obtain a positive electrode slurry; thereafter, the positive electrode slurry was evenly applied on a positive electrode current collector, followed by oven drying, cold pressing and slitting to obtain a positive electrode plate.

### Preparation of negative electrode plate:

A negative electrode active material, a conductive agent carbon black, a binder styrenebutadiene rubber (SBR), and a thickener sodium hydroxymethylcellulose (CMC) were dissolved in a solvent deionized water according to a weight ratio of 90:4:4:2, and then mixed well to form a negative electrode slurry; the negative slurry was uniformly applied once or more times to coat the a copper foil of a negative electrode current collector, followed by oven drying, cold pressing, and cutting to obtain a negative electrode plate with a negative electrode film layer. The porosity of the negative electrode film layer was 40%, and the D_{V}50 of the graphite used was 18 µm.

### Separator:

A conventional polypropylene film was used as the separator.

### Assembly of lithium-ion battery:

The positive electrode plate, the separator and the negative electrode plate were stacked sequentially, wherein the separator was positioned between the positive electrode plate and the negative electrode plate to separate them, and then the positive electrode, the separator and the negative electrode plate were wound to obtain an electrode assembly; the electrode assembly was put into a battery case and dried, and then injected with the electrolyte, followed by processes such as chemical formation and standing to obtain a lithium-ion battery.

The material or amount of the first additive and the material or amount of the second additive in the electrolyte of Examples 1 to 53 and Comparative Examples 1 to 9 were all recorded in Table 3, and the rest was the same as in Example 1.

**Table 3**

| | First additive | Content W1 (%) | Second additive | Content W2 (%) | W1/W2 |
|---|---|---|---|---|---|
| Example 1 | Compound 1 | 2 | Compound 3-6 | 2 | 1 |
| Example 2 | Compound 2 | 2 | Compound 3-6 | 2 | 1 |
| Example 3 | Compound 3 | 2 | Compound 3-6 | 2 | 1 |
| Example 4 | Compound 4 | 2 | Compound 3-6 | 2 | 1 |
| Example 5 | Compound 5 | 2 | Compound 3-6 | 2 | 1 |
| Example 6 | Compound 6 | 2 | Compound 3-6 | 2 | 1 |
| Example 7 | Compound 7 | 2 | Compound 3-6 | 2 | 1 |
| Example 8 | Compound 8 | 2 | Compound 3-6 | 2 | 1 |
| Example 9 | Compound 9 | 2 | Compound 3-6 | 2 | 1 |
| Example 10 | Compound 10 | 2 | Compound 3-6 | 2 | 1 |
| Example 11 | Compound 11 | 2 | Compound 3-6 | 2 | 1 |
| Example 12 | Compound 12 | 2 | Compound 3-6 | 2 | 1 |
| Example 13 | Compound 1 | 2 | Compound 3-7 | 2 | 1 |
| Example 14 | Compound 1 | 2 | Compound 3-8 | 2 | 1 |
| Example 15 | Compound 1 | 2 | Compound 3-1 | 2 | 1 |
| Example 16 | Compound 1 | 2 | Compound 3-4 | 2 | 1 |
| Example 17 | Compound 1 | 2 | Compound 3-3 | 2 | 1 |
| Example 18 | Compound 1 | 2 | Compound 3-9 | 2 | 1 |
| Example 19 | Compound 1 | 2 | Compound 4-2 | 2 | 1 |
| Example 20 | Compound 1 | 2 | Compound 4-5 | 2 | 1 |
| Example 21 | Compound 1 | 2 | Compound 4-6 | 2 | 1 |
| Example 22 | Compound 1 | 2 | Compound 4-8 | 2 | 1 |
| Example 23 | Compound 1 | 2 | Compound 5-1 | 2 | 1 |
| Example 24 | Compound 1 | 2 | Compound 5-2 | 2 | 1 |
| Example 25 | Compound 1 | 2 | Compound 5-5 | 2 | 1 |
| Example 26 | Compound 1 | 2 | Compound 5-6 | 2 | 1 |
| Example 27 | Compound 1 | 2 | Compound 5-7 | 2 | 1 |
| Example 28 | Compound 1 | 2 | Compound 6-1 | 2 | 1 |
| Example 29 | Compound 1 | 2 | Compound 6-3 | 2 | 1 |
| Example 30 | Compound 1 | 2 | Compound 6-4 | 2 | 1 |
| Example 31 | Compound 1 | 2 | Compound 6-5 | 2 | 1 |
| Example 32 | Compound 1 | 2 | Compound 7-1 | 2 | 1 |
| Example 33 | Compound 1 | 2 | Compound 7-2 | 2 | 1 |
| Example 34 | Compound 1 | 2 | Compound 7-4 | 2 | 1 |
| Example 35 | Compound 1 | 2 | Compound 7-6 | 2 | 1 |
| Example 36 | Compound 1 | 2 | Compound 7-8 | 2 | 1 |
| Example 37 | Compound 13 | 2 | Compound 3-8 | 2 | 1 |
| Example 38 | Compound 13 | 2 | Compound 4-2 | 2 | 1 |
| Example 39 | Compound 13 | 2 | Compound 5-2 | 2 | 1 |
| Example 40 | Compound 13 | 2 | Compound 6-1 | 2 | 1 |
| Example 41 | Compound 13 | 2 | Compound 7-2 | 2 | 1 |
| Example 42 | Compound 1 | 0.01 | Compound 3-6 | 2 | 0.005 |
| Example 43 | Compound 1 | 0.1 | Compound 3-6 | 2 | 0.05 |
| Example 44 | Compound 1 | 5 | Compound 3-6 | 2 | 2.5 |
| Example 45 | Compound 1 | 10 | Compound 3-6 | 2 | 5 |
| Example 46 | Compound 1 | 20 | Compound 3-6 | 2 | 10 |
| Example 47 | Compound 1 | 25 | Compound 3-6 | 2 | 12.5 |
| Example 48 | Compound 1 | 0.001 | Compound 3-6 | 0.01 | 0.1 |
| Example 49 | Compound 1 | 0.001 | Compound 3-6 | 0.1 | 0.01 |
| Example 50 | Compound 1 | 2 | Compound 3-6 | 5 | 0.4 |
| Example 51 | Compound 1 | 2 | Compound 3-6 | 10 | 0.2 |
| Example 52 | Compound 1 | 2 | Compound 3-6 | 20 | 0.1 |
| Example 53 | Compound 1 | 2 | Compound 3-6 | 25 | 0.08 |
| Comparative Example 1 | Compound 1 | 2 | None | 0 | - |
| Comparative Example 2 | None | 0 | Compound 15 | 2 | - |
| Comparative Example 3 | None | 0 | Compound 3-6 | 2 | - |
| Comparative Example 4 | None | 0 | Compound 4-2 | 2 | - |
| Comparative Example 5 | None | 0 | Compound 5-2 | 2 | - |
| Comparative Example 6 | None | 0 | Compound 6-1 | 2 | - |
| Comparative Example 7 | None | 0 | Compound 7-2 | 2 | - |
| Comparative Example 8 | Compound 1 | 2 | None | 0 | - |
| Comparative Example 9 | None | 0 | Compound 3-6 | 2 | - |

The porosity of the negative electrode film layer of Examples 54 to 63 and the Dv50 of the negative electrode material used were all recorded in Table 4, and the rest was the same as in Example 1.

**Table 4**

| | Porosity of negative electrode (%) | Negative electrode Dᵥ50(µm) |
|---|---|---|
| Example 54 | 25 | 18 |
| Example 55 | 30 | 18 |
| Example 56 | 37 | 18 |
| Example 57 | 42 | 18 |
| Example 58 | 45 | 18 |
| Example 59 | 48 | 18 |
| Example 60 | 40 | 6 |
| Example 61 | 40 | 15 |
| Example 62 | 40 | 20 |
| Example 63 | 40 | 22 |

### Performance Test:

### 1. Cycling Performance Test

At 25°C, a lithium-ion battery was charged to 3.65 V with a constant current of 0.5 C, and then charged with a constant voltage of 3.65 until the current was less than 0.05 C, and the lithium-ion battery is then discharged to 2.5 V with a constant current of 0.5 C, which was a charge-discharge process. The charging and discharging were repeated as such, and the number of cycles after the lithium-ion battery attenuated to 80% was calculated.

### 2. Storage Performance Test

At 25°C, the prepared lithium-ion secondary battery was first charged to 3.65 V with a constant current of 0.33 C, and further charged to a current of 0.05 C with a constant voltage of 3.65 V, and the lithium-ion secondary battery was then discharged to 2.5 V with a constant current of 0.33 C. The discharge capacity C0 at this time was a discharge capacity of the lithium-ion secondary battery before high-temperature storage; then the lithium-ion secondary battery was charged to 3.65 V with a constant current of 0.33 C, and charged to a current of 0.05 C with a constant voltage of 3.65 V to fully charge the lithium-ion battery. The battery was placed in a 60°C oven for 30 days, the battery was then taken out, and the battery was put in a 25°C environment for 0.33 C discharging. The discharge capacity was recorded as C1; capacity retention rate = (C1/C0) × 100%

### 3. Volume Expansion Rate Test

At 25°C, the prepared lithium-ion secondary battery was first charged to 3.65 V with a constant current of 0.33 C, and further charged to a current of 0.05 C with a constant voltage of 3.65 V, and the lithium-ion secondary battery was then discharged to 2.5 V with a constant current of 0.33 C. The discharge capacity at this time was a discharge capacity of the lithium-ion secondary battery before high-temperature storage; then the lithium-ion secondary battery was charged to 3.65 V with a constant current of 0.33 C, and charged to a current of 0.05 C with a constant voltage of 3.65 V to fully charge the lithium-ion battery. The volume of the battery was tested using a water displacement method. Thereafter, the lithium-ion battery was stored at 60°C for 60 days. After the storage ended, the lithium-ion secondary battery was put in a 25°C environment, and the volume of the battery was tested using the water displacement method. Battery volume expansion rate = (volume after storage/volume before storage - 1)%.

The test results are recorded in Table 5.

**Table 5**

| | Cycling Performance (cycles) | Storage Performance | Volume Expansion Rate |
|---|---|---|---|
| Example 1 | 3209 | 92.1% | 3.30% |
| Example 2 | 3096 | 91.5% | 3.70% |
| Example 3 | 2991 | 91.0% | 3.90% |
| Example 4 | 3007 | 91.3% | 3.60% |
| Example 5 | 2984 | 91.4% | 4.30% |
| Example 6 | 2819 | 89.3% | 5.50% |
| Example 7 | 2759 | 87.5% | 6.80% |
| Example 8 | 2693 | 87.6% | 6.70% |
| Example 9 | 2783 | 89.1% | 6.90% |
| Example 10 | 2571 | 86.7% | 7.20% |
| Example 11 | 2746 | 85.9% | 7.30% |
| Example 12 | 3104 | 91.7% | 3.50% |
| Example 13 | 2909 | 90.1% | 3.78% |
| Example 14 | 2891 | 89.3% | 3.82% |
| Example 15 | 2803 | 89.7% | 4.12% |
| Example 16 | 3112 | 90.5% | 3.46% |
| Example 17 | 3104 | 91.5% | 3.45% |
| Example 18 | 2765 | 92.1% | 3.35% |
| Example 19 | 2809 | 89.3% | 4.41% |
| Example 20 | 2876 | 89.5% | 4.32% |
| Example 21 | 2941 | 89.7% | 4.11% |
| Example 22 | 2753 | 88.7% | 4.73% |
| Example 23 | 3052 | 91.3% | 3.78% |
| Example 24 | 2809 | 90.2% | 4.25% |
| Example 25 | 3009 | 91.1% | 3.81% |
| Example 26 | 2855 | 90.7% | 4.18% |
| Example 27 | 2741 | 88.9% | 4.31% |
| Example 28 | 2871 | 89.4% | 3.95% |
| Example 29 | 2711 | 88.5% | 4.75% |
| Example 30 | 2703 | 88.3% | 4.82% |
| Example 31 | 2841 | 90.1% | 3.87% |
| Example 32 | 2809 | 89.8% | 3.92% |
| Example 33 | 2758 | 88.7% | 4.25% |
| Example 34 | 2832 | 88.9% | 4.06% |
| Example 35 | 2901 | 89.6% | 3.92% |
| Example 36 | 2954 | 89.8% | 3.85% |
| Example 37 | 2997 | 90.1% | 3.82% |
| Example 38 | 2984 | 89.6% | 3.93% |
| Example 39 | 2887 | 89.2% | 4.12% |
| Example 40 | 2704 | 88.7% | 4.35% |
| Example 41 | 2704 | 88.5% | 4.55% |
| Example 42 | 2541 | 86.5% | 6.82% |
| Example 43 | 2711 | 88.7% | 5.92% |
| Example 44 | 3182 | 91.8% | 3.35% |
| Example 45 | 3121 | 91.4% | 3.37% |
| Example 46 | 2901 | 89.4% | 4.87% |
| Example 47 | 2781 | 88.7% | 5.13% |
| Example 48 | 2441 | 86.1% | 7.52% |
| Example 49 | 2481 | 86.2% | 7.22% |
| Example 50 | 3053 | 90.2% | 3.61% |
| Example 51 | 3011 | 90.0% | 3.75% |
| Example 52 | 2913 | 89.2% | 4.21% |
| Example 53 | 2719 | 88.9% | 4.54% |
| Example 54 | 2611 | 87.2% | 6.14% |
| Example 55 | 2745 | 88.3% | 5.74% |
| Example 56 | 2971 | 90.1% | 4.21% |
| Example 57 | 3011 | 90.4% | 4.09% |
| Example 58 | 2852 | 89.8% | 4.36% |
| Example 59 | 2712 | 87.9% | 5.11% |
| Example 60 | 2877 | 89.4% | 4.41% |
| Example 61 | 2989 | 90.3% | 4.01% |
| Example 62 | 3109 | 91.7% | 3.47% |
| Example 63 | 2909 | 91.1% | 3.66% |
| Comparative Example 1 | 2491 | 87.2% | 7.68% |
| Comparative Example 2 | 2354 | 83.5% | 5.11% |
| Comparative Example 3 | 2367 | 87.8% | 6.25% |
| Comparative Example 4 | 2297 | 87.1% | 5.82% |
| Comparative Example 5 | 2284 | 86.4% | 5.63% |
| Comparative Example 6 | 2187 | 85.3% | 6.12% |
| Comparative Example 7 | 2004 | 84.6% | 8.35% |

According to data comparison in Table 5, it can be found that when different first additives are used in combination with the same second additive, both the cycling performance and storage performance of the battery are improved, but the degree of improvement is different. Moreover, according to the data comparison for Example 1 and Examples 42 to 53, it can be found that the amounts of the first additives and the second additive and the ratio between the two affect the improvement in the cycling performance and storage performance of the battery, especially when W1/W2 is between 0.01 and 1, the cycling performance, storage performance, and volume expansion rate of the battery are all significantly improved. According to the data comparison for Examples 54 to 63, it can be found that the porosity of the negative electrode film layer on the negative electrode side and the Dᵥ50 of the negative electrode active material have an impact on the improvement in the cycling performance, storage performance, and volume expansion rate of the battery.

Although the present application has been described with reference to the preferred embodiments, various improvements can be made thereto and components thereof can be replaced with equivalents without departing from the scope of the present application. In particular, the technical features mentioned in the various embodiments can be combined in any manner as long as there is no structural conflict. The present application is not limited to the particular embodiments disclosed herein, but rather includes all technical solutions falling within the scope of the claims.

## Claims

1. A non-aqueous electrolyte, comprising additives, wherein the additives comprise a first additive and a second additive; the first additive is any one or more cyclic sulfate compounds having a structure represented by general formula (I), 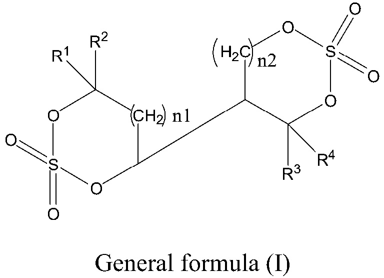
wherein R¹, R², R³ and R⁴ are each independently selected from any one of a group having a structure represented by general formula (II), a hydrogen atom, a halogen atom, a C1-C6 alkyl group, a C1-C6 haloalkyl group, a C1-C6 alkoxy group, a C1-C6 haloalkoxy group, a C2-C6 alkenyl group, a C2-C6 ester group, a cyano group, and a sulfonic acid group, and n1 and n2 are each independently any integer of 0-2,
the general formula (II) is 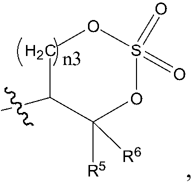
wherein R⁵ and R⁶ are each independently selected from any one of a group having a structure represented by the general formula (II), a hydrogen atom, a halogen atom, a C1-C6 alkyl group, a C1-C6 haloalkyl group, a C1-C6 alkoxy group, a C1-C6 haloalkoxy group, a C2-C6 alkenyl group, a C2-C6 ester group, a cyano group, and a sulfonic acid group, and n3 is any integer of 0-2; and
the second additive is an organic base additive; the organic base additive comprises any one or more of groups consisting of 5-12 membered aromatic heterocyclic organic bases or 5-12 membered aliphatic heterocyclic organic bases, and ring structures of the 5-12 membered aromatic heterocyclic organic bases and the 5-12 membered aliphatic heterocyclic organic bases contain nitrogen atoms.

2. The non-aqueous electrolyte of claim 1, wherein R¹ and R² are not both hydrogen atoms, and R³ and R⁴ are not both hydrogen atoms;
alternatively, R¹, R², R³, R⁴, R⁵ and R⁶ satisfy the following conditions:
R¹ and R² are both hydrogen atoms, one of R³ and R⁴ is a hydrogen atom while the other is any one of a group having a structure represented by the general formula (II), a halogen atom, a C1-C6 alkyl group, a C1-C6 haloalkyl group, a C1-C6 alkoxy group, a C1-C6 haloalkoxy group, a C2-C6 alkenyl group, a C2-C6 ester group, a cyano group, and a sulfonic acid group, and R⁵ and R⁶ in the group having the structure represented by the general formula (II) are not both hydrogen atoms;
alternatively, R¹, R², R³, R⁴, R⁵ and R⁶ satisfy the following conditions:
R³ and R⁴ are both hydrogen atoms, one of R¹ and R² is a hydrogen atom while the other is any one of a group having a structure represented by the general formula (II), a halogen atom, a C1-C6 alkyl group, a C1-C6 haloalkyl group, a C1-C6 alkoxy group, a C1-C6 haloalkoxy group, a C2-C6 alkenyl group, a C2-C6 ester group, a cyano group, and a sulfonic acid group, and R⁵ and R⁶ in the group having the structure represented by the general formula (II) are not both hydrogen atoms.

3. The non-aqueous electrolyte of claim 1 or 2, wherein the cyclic sulfate compound has a structure represented by general formula (I-1), 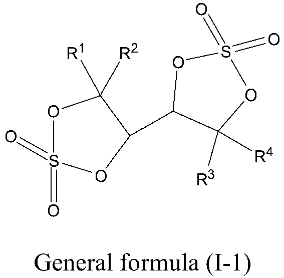
wherein R¹, R², R³ and R⁴ are each independently selected from any one of a group having a structure represented by general formula (II-1), a hydrogen atom, a halogen atom, a C1-C6 alkyl group, a C1-C6 haloalkyl group, a C1-C6 alkoxy group, a C1-C6 haloalkoxy group, a C2-C6 alkenyl group, a C2-C6 ester group, a cyano group, and a sulfonic acid group; and
the general formula (II-1) is 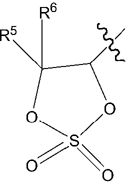
wherein R⁵ and R⁶ are each independently selected from any one of a group having a structure represented by the general formula (II-1), a hydrogen atom, a halogen atom, a C1-C6 alkyl group, a C1-C6 haloalkyl group, a C1-C6 alkoxy group, a C1-C6 haloalkoxy group, a C2-C6 alkenyl group, a C2-C6 ester group, a cyano group, and a sulfonic acid group.

4. The non-aqueous electrolyte of any one of claims 1 to 3, wherein
R¹, R², R³, R⁴, R⁵ and R⁶ are each independently selected from any one of a group having a structure represented by the general formula (II-1), a hydrogen atom, a halogen atom, a C1-C3 alkyl group, a C1-C3 haloalkyl group, a C1-C3 alkoxy group, a C1-C3 haloalkoxy group, a C2-C3 alkenyl group, a C1-C3 ester group, a cyano group, and a sulfonic acid group;
optionally, R¹, R², R³, R⁴, R⁵ and R⁶ are each independently selected from any one of a group having a structure represented by the general formula (II-1), a hydrogen atom, a halogen atom, a C1-C3 alkyl group and a C1-C3 haloalkyl group;
optionally, R¹, R², R³, R⁴, R⁵ and R⁶ are each independently selected from any one of a group having a structure represented by the general formula (II-1), a hydrogen atom, an F atom, a Cl atom, a Br atom, a methyl group, an ethyl group, a propyl group and an isopropyl group;
optionally, the group having the structure represented by the general formula (II-1) is selected from any one of the following groups: 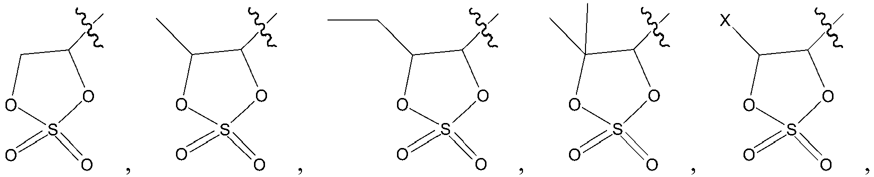 and 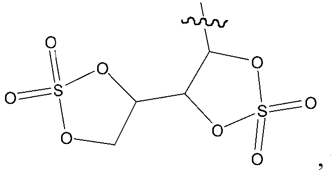 wherein X is an F atom, a Cl atom, or a Br atom;
optionally, R¹, R², R³ and R⁴ are each independently selected from 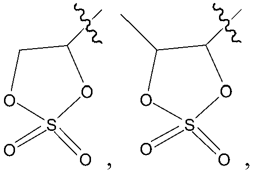 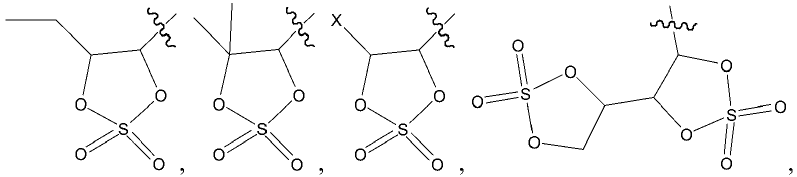 a hydrogen atom, an F atom, a Cl atom, a Br atom, a methyl group, an ethyl group, a propyl group, and an isopropyl group, and X is an F atom; and
further optionally, R¹, R², R³ and R⁴ are each independently selected from 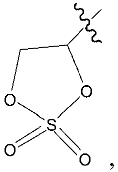 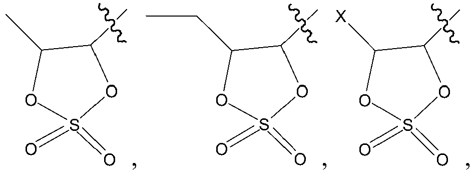 a hydrogen atom, a methyl group, and an ethyl group, and X is an F atom.

5. The non-aqueous electrolyte of claim 1, wherein the cyclic sulfate compound is selected from one or more of the following compounds:

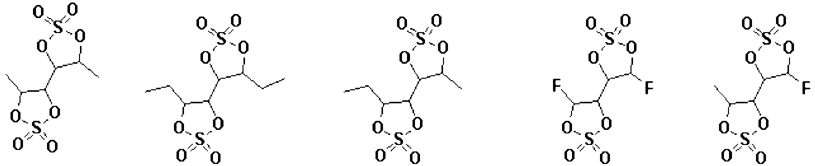

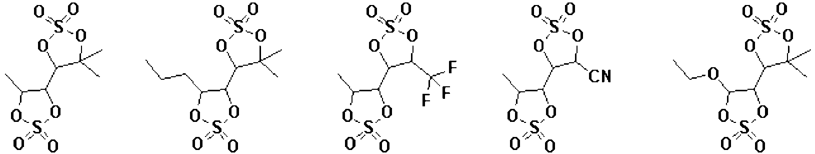

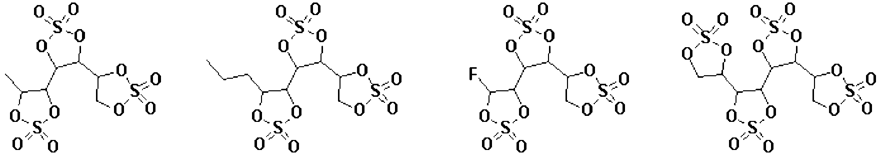

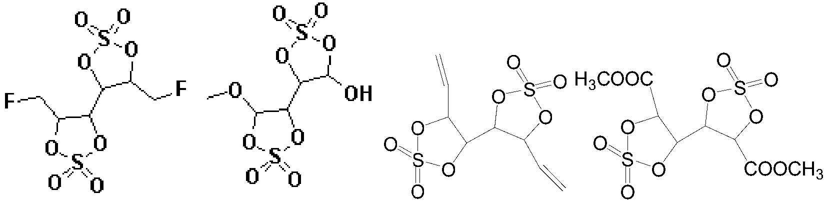

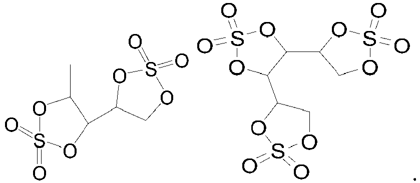

6. The non-aqueous electrolyte of any one of claims 1 to 5, wherein the 5-12 membered aromatic heterocyclic organic bases comprise one or more selected from the group consisting of a compound having a structure represented by general formula (III), a compound having a structure represented by general formula (IV), and a compound having a structure represented by general formula (V), wherein 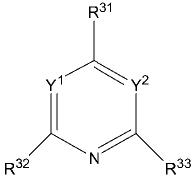 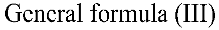 in the general formula (III), Y¹and Y² are each independently a C or N element; R³¹, R³²and R³³ are each independently selected from any one of a hydrogen atom, a halogen atom, a C1-C6 alkyl group, a C2-C6 alkenyl group, a C2-C6 alkynyl group, -R³⁴OH, -R³⁵N R³⁶R³⁷ and-R³⁸-OR³⁹; R³⁴, R³⁵ and R³⁸ are each independently selected from any one of a C0-C6 alkylene group and a C2-C6 alkenylene group; R³⁶, R³⁷ and R³⁹ are each independently selected from any one of a hydrogen atom, a halogen atom, a C1-C6 alkyl group and a C1-C6 haloalkyl group; any carbon atom in the C1-C6 alkyl group as the R³⁶, R³⁷ and R³⁹ is optionally substituted with a heteroatom; the heteroatom is an N atom, an S atom or a P atom; and optionally, the R³⁶ and R³⁷ are connected to form a ring; 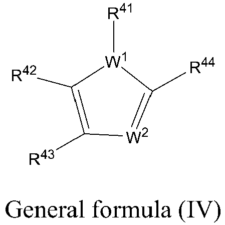 in the general formula (IV), W¹ is C, N, O or S; W² is C or N; at least one of W¹ and W² is N; R⁴¹, R⁴², R⁴³ and R⁴⁴ are each independently selected from any one of a hydrogen atom, a halogen atom, a C1-C6 alkyl group, a C2-C6 alkenyl group, a C2-C6 alkynyl group, -R⁴⁵OH, - R⁴⁶N R⁴⁷R⁴⁸ and -R⁴⁹-O-R⁵⁰; R⁴⁵, R⁴⁶ and R⁴⁹ are each independently selected from any one of a C0-C6 alkylene group and a C2-C6 alkenylene group; and R⁴⁷, R⁴⁸ and R⁵⁰ are each independently selected from any one of a hydrogen atom, a halogen atom, a C1-C6 alkyl group and a C1-C6 haloalkyl group; and 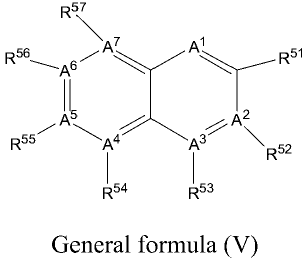 in the general formula (V), A¹, A², A³, A⁴, A⁵, A⁶ and A⁷ are each independently C or N; R⁵¹, R⁵², R⁵³, R⁵⁴, R⁵⁵, R⁵⁶ and R⁵⁷ are any one of a hydrogen atom, a C1-C6 alkyl group, a C2-C6 alkenyl group, a C2-C6 alkynyl group, -OH, -R⁵⁸N R⁵⁹R⁶⁰ and an alkoxy group; R⁵⁸is selected from any one of a C0-C6 alkylene group and a C2-C6 alkenylene group; and R⁵⁹ and R⁶⁰ are each independently selected from any one of a hydrogen atom, a halogen atom, a C1-C6 alkyl group and a C1-C6 haloalkyl group.

7. The non-aqueous electrolyte of claim 6, wherein in general formula (III), R³¹, R³² and R³³ are each independently selected from any one of a hydrogen atom, a halogen atom, a C1-C4 alkyl group, a C3-C5 alkenyl group, a C3-C5 alkynyl group, -R³⁴OH, -R³⁵N R³⁶R³⁷ and -R³⁸-O-R³⁹. R³⁴, R³⁵ and R³⁸are each independently selected from any one of C0-C3 alkylene groups; R³⁶, R³⁷ and R³⁹ are each independently selected from any one of a hydrogen atom, a halogen atom, a C1-C3 alkyl group and a C1-C3 haloalkyl group; any carbon atom in the C1-C6 alkyl group as the R³⁶, R³⁷ and R³⁹ is optionally substituted with a heteroatom; the heteroatom is an N atom; and optionally, the R³⁶ and R³⁷ are connected to form a 5-membered or 6-membered aliphatic heterocycle;
optionally, R³¹, R³² and R³³ are each independently selected from any one of a hydrogen atom, a halogen atom, a methyl group, an ethyl group, an n-propyl group, an isopropyl group, an allyl group, a propargyl group, -OH, -CH₃OH, -NH₂, -CH₂NH₂, -N(CH₃)₂, O-CH₃ and 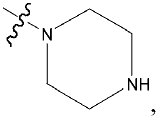 and optionally, the R³¹, R³² and R³³ are each independently selected from any one of a hydrogen atom, an F atom, a methyl group, an ethyl group, an n-propyl group, an isopropyl group, -CH₃OH, -CH₂NH₂, -N(CH₃)₂, O-CH₃ and 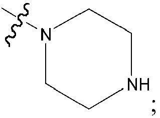 and
optionally, the compound having the structure represented by general formula (III) is selected from any one or more of the following compounds:

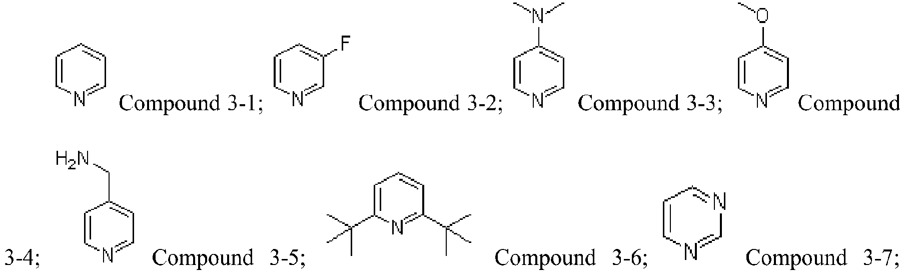

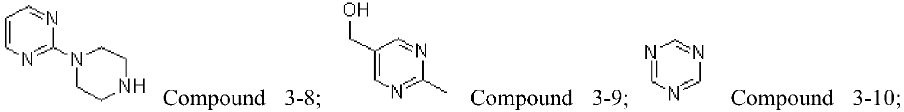

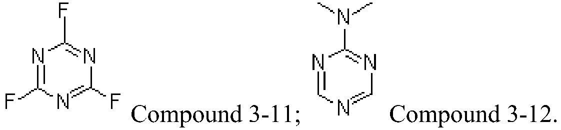

8. The non-aqueous electrolyte of claim 6, wherein in the general formula (IV), W¹ is N, and W² is C or N;
optionally, R⁴¹, R⁴², R⁴³ and R⁴⁴ are each independently selected from any one of a hydrogen atom, a halogen atom, a C1-C4 alkyl group, a C3-C5 alkenyl group, a C3-C5 alkynyl group, - W⁴⁴OH, -R⁴⁵N R⁴⁶R⁴⁷, and -R⁴⁸-O-R⁴⁹; R⁴⁴, R⁴⁵ and R⁴⁸ are each independently selected from any one of C0-C4 alkylene groups; and R⁴⁶, R⁴⁷ and R⁴⁹ are each independently selected from any one of a hydrogen atom, a halogen atom, a C1-C4 alkyl group and a C1-C4 haloalkyl group;
optionally, R⁴¹, R⁴², R⁴³ and R⁴⁴ are each independently selected from any one of a hydrogen atom, an F atom, a methyl group, an ethyl group, a propyl group, a cyclopropyl group, an allyl group, a propargyl group, -OH, -CH₃OH, -NH₂, -NHCH₃, -CH₂NH₂, -N(CH₃)₂ and O-CH₃;
optionally, R⁴¹, R⁴², R⁴³ and R⁴⁴ are each independently selected from any one of a hydrogen atom, an F atom, a methyl group, a propyl group, a cyclopropyl group, an allyl group, -CH₃OH, - NH₂, -NHCH₃ and -N(CH₃)₂; and
optionally, the compound having the structure represented by general formula (IV) is selected from any one or more of the following compounds:

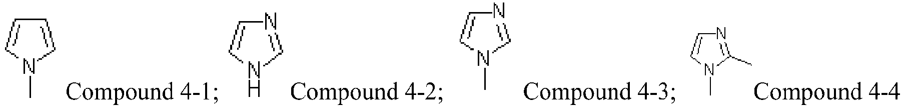

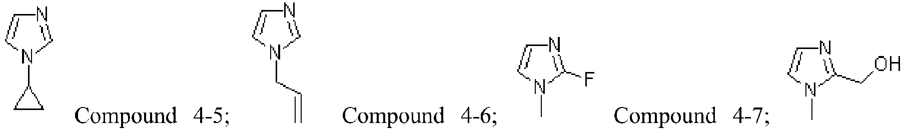

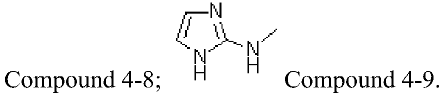

9. The non-aqueous electrolyte of claim 6, wherein in the general formula (V), A¹ is N, and A², A³, A⁴, A⁵, A⁶ and A⁷ are each independently C or N;
optionally, R⁵¹, R⁵², R⁵³, R⁵⁴, R⁵⁵, R⁵⁶ and R⁵⁷ are any one of a hydrogen atom, a C1-C4 alkyl group, a C2-C4 alkenyl group, a C2-C4 alkynyl group, -OH, -R⁵⁸N R⁵⁹R⁶⁰ and an alkoxy group; R⁵⁸ is selected from any one of C0-C3 alkylene groups; and R⁵⁹ and R⁶⁰ are each independently selected from any one of a hydrogen atom, a halogen atom, a C1-C3 alkyl group and a C1-C3 haloalkyl group;
optionally, R⁵¹, R⁵², R⁵³, R⁵⁴, R⁵⁵, R⁵⁶ and R⁵⁷ are any one of a hydrogen atom, a methyl group, an ethyl group, an allyl group, a propargyl group, -OH, -NH₂, -CH₂NH₂, -N(CH₃)₂ and O-CH₃;
optionally, R⁵¹, R⁵², R⁵³, R⁵⁴, R⁵⁵, R⁵⁶ and R⁵⁷ are any one of a hydrogen atom, a methyl group, an ethyl group, and O-CH₃; and
optionally, the compound having the structure represented by general formula (V) is selected from any one or more of the following compounds:

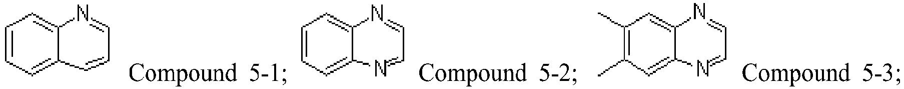

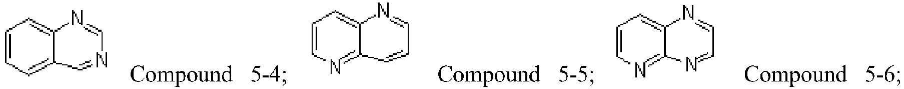

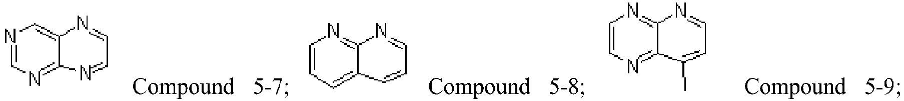

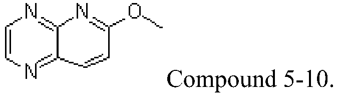

10. The non-aqueous electrolyte of any one of claims 1 to 9, wherein the 5-12 membered aliphatic heterocyclic organic bases comprise one or more selected from the group consisting of a compound having a structure represented by general formula (VI) and a compound having a structure represented by general formula (VII), wherein 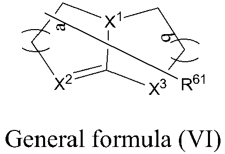 in the general formula (VI), X¹, X² and X³ are each independently C or N and at least one of them is required to be N; a and b are each independently an integer of 0-3; each R⁶¹ is independently selected from any one of a hydrogen atom, a halogen atom, a C1-C6 alkyl group, a C2-C6 alkenyl group, a C2-C6 alkynyl group, -R⁶²OH, -R⁶³N R⁶⁴R⁶⁵ and -R⁶⁶-O-R⁶⁷; R⁶², R⁶³ and R⁶⁴ are each independently selected from any one of a C0-C6 alkylene group and a C2-C6 alkenylene group; and R⁶⁵, R⁶⁶ and R⁶⁷ are each independently selected from any one of a hydrogen atom, a halogen atom, a C1-C6 alkyl group and a C1-C6 haloalkyl group, and 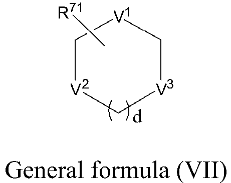 in the general formula (VI), V¹, V² and V³ are each independently C or N and at least one of them is required to be N; d is an integer of 0-3; each R⁷¹ is independently selected from any one of a hydrogen atom, a halogen atom, a C1-C6 alkyl group, a C2-C6 alkenyl group, a C2-C6 alkynyl group, -R⁷²OH, -R⁷³N R⁷⁴R⁷⁵ and -R⁷⁶-O-R⁷⁷; R⁷², R⁷³ and R⁷⁴ are each independently selected from any one of a C0-C6 alkylene group and a C2-C6 alkenylene group; and R⁷⁵, R⁷⁶ and R⁷⁷ are each independently selected from any one of a hydrogen atom, a halogen atom, a C1-C6 alkyl group and a C1-C6 haloalkyl group.

11. The non-aqueous electrolyte of claim 10, wherein in the general formula (VI), X¹ is N, and X² and X³ are each independently C or N;
optionally, a and b are each independently 0, 1 or 2;
optionally, each R⁶¹ is independently selected from any one of a hydrogen atom, a halogen atom, a C1-C4 alkyl group, a C3-C5 alkenyl group, a C3-C5 alkynyl group, -R⁶²OH, -R⁶³N R⁶⁴R⁶⁵, and -R⁶⁶-O-R⁶; R⁶², R⁶³ and R⁶⁴ are each independently selected from any one of C0-C3 alkylene groups; and R⁶⁵, R⁶⁶ and R⁶⁷ are each independently selected from any one of a hydrogen atom, a halogen atom, a C1-C4 alkyl group and a C1-C4 haloalkyl group;
optionally, each R⁶¹ is independently selected from any one of a hydrogen atom, an F atom, a methyl group, an ethyl group, a hydroxyl group, -NH₂, and -N(CH₃)₂; and
optionally, the compound having the structure represented by the general formula (VI) is selected from any one or more of the following compounds:

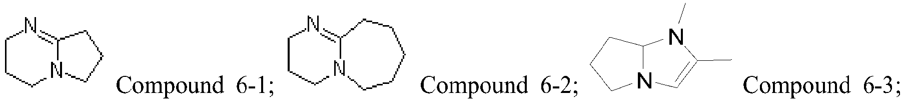

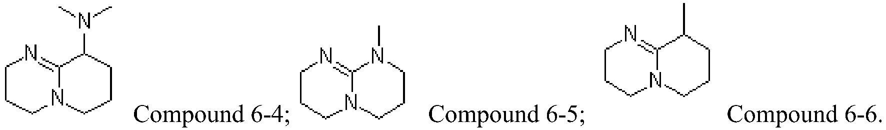

12. The non-aqueous electrolyte of claim 10, wherein in the general formula (VII), d is 0 or 1;
optionally, each R⁷¹ is independently selected from any one of a hydrogen atom, a halogen atom, a C1-C4 alkyl group, a C3-C5 alkenyl group, a C3-C5 alkynyl group, -R⁷²OH, -R⁷³N R⁷⁴R⁷⁵, and -R⁷⁶-O-R⁷⁷; R⁷², R⁷³ and R⁷⁴ are each independently selected from any one of C0-C3 alkylene groups; and R⁷⁵, R⁷⁶ and R⁷⁷ are each independently selected from any one of a hydrogen atom, a halogen atom, a C1-C4 alkyl group and a C1-C4 haloalkyl group;
optionally, each R⁷¹ is independently selected from any one of a hydrogen atom, an F atom, a methyl group, an ethyl group, an n-propyl group, an isopropyl group, a hydroxyl group, -NH₂, and -N(CH₃)₂;
optionally, each R⁷¹ is independently selected from any one of a hydrogen atom, an F atom, a methyl group, an ethyl group, an n-propyl group, and an isopropyl group; and
optionally, the compound having the structure represented by general formula (VII) is selected from any one or more of the following compounds:

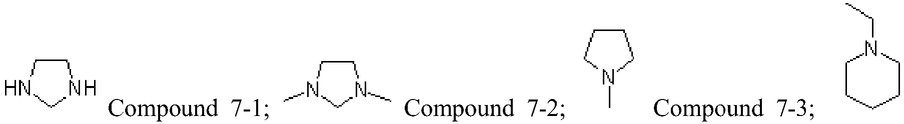

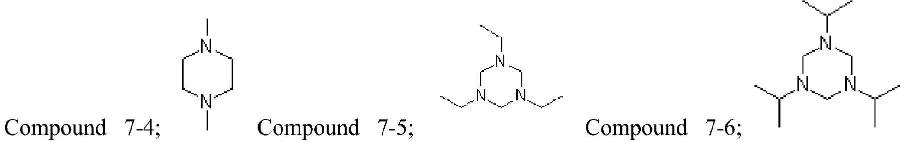

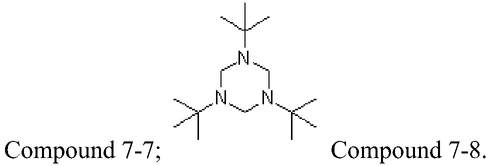

13. The non-aqueous electrolyte of any one of claims 1 to 12, wherein mass percent of the first additive in the non-aqueous electrolyte is W1, W1 is optionally 0.001-20%, and W1 is further optionally 0.1-5%;
and/or mass percent of the second additive in the non-aqueous electrolyte is W2, W2 is optionally 0.001-20%, and W2 is further optionally 0.1-5%; and
optionally, 0.01≤W1/W2≤10, preferably 0.05≤W1/W2≤5.

14. The non-aqueous electrolyte of any one of claims 1 to 13, wherein the non-aqueous electrolyte further comprises an electrolyte; the electrolyte optionally comprises an alkali metal salt electrolyte; the electrolyte optionally comprises a lithium salt or a sodium salt; the lithium salt optionally comprises one or more selected from the group consisting of lithium hexafluorophosphate, lithium perchlorate, lithium hexafluoroarsenate, lithium bis(fluorosulfonyl)imide, and lithium bis(trifluoromethanesulfonyl)imide; the sodium salt comprises one or more selected from the group consisting of sodium hexafluorophosphate, sodium difluoro(oxalato)borate, sodium perchlorate, sodium bis(fluorosulfonyl)imide, sodium bis(trifluoromethanesulfonyl)imide, and sodium trifluoromethanesulfonate.

15. The non-aqueous electrolyte of any one of claims 1 to 14, wherein the non-aqueous electrolyte further comprises a non-aqueous solvent; the non-aqueous solvent optionally comprises any one or more selected from the group consisting of cyclic carbonate, chain carbonate, a nitrile solvent, a ketone solvent, and a sulfone solvent; and the non-aqueous solvent further optionally comprises one or more selected from the group consisting of ethylene carbonate, propylene carbonate, methyl ethyl carbonate, diethyl carbonate, dimethyl carbonate, dipropyl carbonate, methyl propyl carbonate, ethyl propyl carbonate, butylene carbonate, fluoroethylene carbonate, methyl formate, methyl acetate, ethyl acetate, propyl acetate, methyl propionate, ethyl propionate, propyl propionate, methyl butyrate, ethyl butyrate, 1,4-butyrolactone, sulfolane, dimethyl sulfone, methyl ethyl sulfone, diethyl sulfone, tetrahydrofuran, glycol dimethyl ether, dioxolane, acetone, acetonitrile, and butyronitrile.

16. The non-aqueous electrolyte of any one of claims 1 to 15, wherein the additives further comprise one or more selected from the group consisting of a sulfate compound, a sulfite compound, a sultone compound, a disulfonic acid compound, a nitrile compound, an aromatic compound, an isocyanate compound, a phosphazene compound, a cyclic acid anhydride compound, a phosphite compound, a phosphate compound, a borate compound, and a carboxylate compound.

17. A secondary battery, comprising a positive electrode plate, an electrolyte, a separator, and a negative electrode plate, wherein the electrolyte comprises the non-aqueous electrolyte of any one of claims 1 to 16, optionally, the secondary battery is a lithium-ion secondary battery or a sodium-ion secondary battery.

18. The secondary battery of claim 17, wherein the negative electrode plate comprises a negative electrode current collector and a negative electrode active material layer arranged on one side or both sides of the negative electrode current collector, and the negative electrode active material layer has a porosity of 30-45%, optionally 37-42%; and
the negative electrode active material layer comprises a negative electrode active material; optionally, the negative electrode active material has Dᵥ50≥6 µm; further optionally, the negative electrode active material has Dᵥ50 between 15 µm and 20 µm.

19. An electrical apparatus, comprising a secondary battery, wherein the secondary battery comprises the secondary battery of any one of claims 17 and 18.
